# FASCICULE DE BREVET EUROPEEN

(11) **EP 0 925 295 B1**
(45) Date de publication et mention de la délivrance du brevet: **04.10.2001**
(21) Numéro de dépôt: 97935612.8
(22) Date de dépôt: 23.07.1997
(51) Int. Cl.: C07D 401/12, A61K 31/47, C07D 401/14

(54) **DERIVES DE N-BENZENESULFONYL-L-PROLINE EN TANT QU' ANTAGONISTES DU RECEPTEUR B2 DE LA BRADYKININE**
N-BENZENSULFONYL-L-PROLINDERIVATE ALS BRADYKININREZEPTOR B2 ANTAGONISTEN
N-BENZENESULPHONYL-L-PROLINE DERIVATIVES AS B2 RECEPTOR ANTAGONISTS OF THE BRADYKININ

(30) Priorité: 24.07.1996 FR 9609327
(43) Date de publication de la demande: 30.06.1999
(73) Titulaire: FOURNIER INDUSTRIE ET SANTE, F-75008 Paris (FR)
(72) Inventeur: DODEY, Pierre, F-21121 Fontaine-lès-Dijon (FR); BONDOUX, Michel, F-21121 Fontaine-lès-Dijon (FR); HOUZIAUX, Patrick, F-78580 Bazemont (FR); BARTH, Martine, F-78490 Montfort-l'Amaury (FR); OU, Khan, F-21121 Hauteville-lès-Dijon (FR)
(74) Mandataire: Hubert, Philippe
(86) Numéro de dépôt international: FR9701377
(87) Numéro de publication internationale: WO9803503

(56) Documents cités:
- EP-A- 0 622 361
- WO-A-96/13485
- WO-A-96/40639
- WO-A-97/07115
- WO-A-97/24349

## Description

### Domaine de l'invention

La présente invention concerne de nouveaux composés dérivés de la N-benzènesulfonyl-L-proline, leur procédé de préparation et leur utilisation en thérapeutique.

Ces nouveaux composés présentent une action inhibitrice vis-à-vis de la bradykinine et sont utiles en thérapeutique, particulièrement pour le traitement de la douleur et de l'inflammation, et notamment dans le traitement de l'asthme, du choc traumatique cérébral et des rhinites allergiques.

### Art antérieur

On sait que l'une des possibilités de traitement de certaines pathologies à caractère douloureux et/ou inflammatoire (telles que l'asthme, la rhinite, le choc septique, la douleur dentaire, etc...) est d'inhiber l'action de certaines hormones telles que la bradykinine ou la kallidine. En effet, ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour créer des composés susceptibles d'être antagonistes des récepteurs de la bradykinine. La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui interagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCI)] ou encore le NPC 17761 (de la société Scios-Nova) présentent une action inhibitrice de la fixation de la bradykinine sur son récepteur B₂. Plus récemment, des composés non peptidiques ont été proposés comme antagonistes vis-à-vis de la fixation de la bradykinine sur son récepteur B₂, notamment dans EP-A-0596406 et EP-A-0622361. On sait en outre que certains composés de structure apparentée à celles des composés visés dans les deux demandes de brevet précitées ont déjà été décrits, notamment dans DE-A-3617183 et EP-A-0261539, eu égard à leurs éventuelles propriétés antithrombotiques.

### But de l'invention

Il existe un besoin d'atténuer ou de supprimer chez les mammifères et surtout chez l'homme les douleurs et les inflammations.

Pour satisfaire ce besoin, on a recherché une nouvelle solution technique qui soit efficace dans le traitement des inflammations et des algies quelle que soit leur origine, notamment dans le traitement des algies liées à des phénomènes inflammatoires.

Selon l'invention, on se propose de fournir une nouvelle solution technique, qui met en oeuvre une fixation compétitive au niveau du récepteur B₂ de la bradykinine entre (i) la bradykinine et les hormones apparentées ou analogues, et (ii) une substance antagoniste, et qui fait appel à des composés de benzènesulfonamide structurellement différents des produits connus précités et qui limitent ou inhibent substantiellement la fixation de la bradykinine et des hormones analogues sur ledit récepteur B₂ de la bradykinine.

Conformément à cette nouvelle solution technique on se propose de fournir, selon un premier aspect de l'invention, des composés dérivés de N-benzènesulfonyl-L-proline en tant que produits industriels nouveaux ; selon un second aspect de l'invention, un procédé de préparation de ces composés; et selon un troisième aspect de l'invention, une utilisation de ces composés notamment en thérapeutique en tant qu'agents antalgiques et/ou anti-inflammatoires.

### Objet de l'invention

Selon la nouvelle solution technique de l'invention, on préconise en tant que produit industriel nouveau, un composé dérivé de la N-benzènesulfonyl-L-proline qui est caractérisé en ce qu'il est choisi parmi l'ensemble constitué par :
(**i**) les composés de formule : 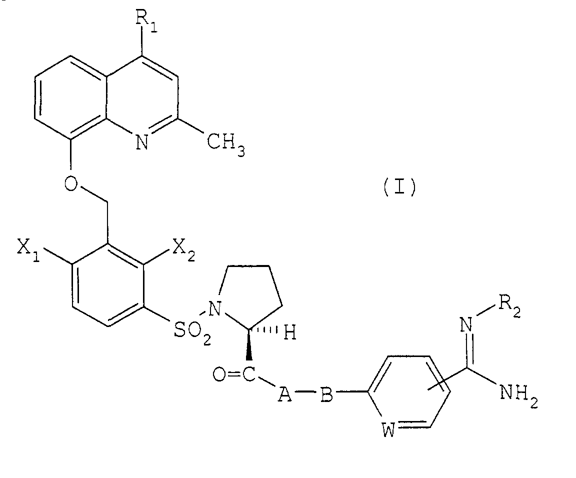 dans laquelle :
   - X₁ et X₂: représentent le même atome d'halogène,
   - R₁: représente un atome d'hydrogène ou un groupe alkyle en C₁-C₃ à chaîne hydrocarbonée linéaire ou ramifiée,
   - R2: représente un atome d'hydrogène,
   - A: représente un groupe 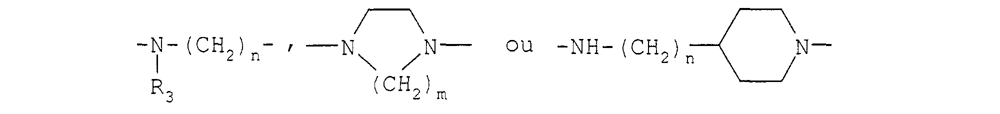
   - B: représente une liaison simple, -CO-, -CO-CH₂-, ou -CO-CH₂-O-,
   - m: représente 2,
   - n: représente 0, 1, 2 ou 3,
   - R₃: représente un atome d'hydrogène ou un groupe méthyle,
   - W: représente CH,
   le groupe amidine C(=NR₂)NH₂ peut être en position 2, 3 ou 4 sur le noyau aromatique, et,
(**ii**) leurs sels d'addition.

Selon l'invention, on préconise aussi un procédé de préparation des composés de formule I et de leurs sels d'addition.

On préconise également l'utilisation d'une substance antagoniste d'un récepteur de la bradykinine et des hormones analogues, ladite utilisation étant caractérisée en ce que l'on fait appel à une substance antagoniste du récepteur B₂ de la bradykinine et choisie parmi les composés de formule I et leurs sels d'addition non-toxiques, pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

### Description détaillée de l'invention

Dans la formule générale I des composés de l'invention, on entend par atome d'halogène un atome de fluor, de chlore, de brome ou d'iode, l'halogène préféré étant l'atome de chlore.

Par groupe alkyle en C₁-C₃ à chaîne hydrocarbonée linéaire ou ramifiée, on entend ici les groupes méthyle, éthyle, propyle et 1-méthyléthyle.

Dans le composé de formule I, l'hétérocycle azoté de structure pyrrolidine comprend 1 atome de carbone asymétrique. Selon l'invention, ce carbone est de configuration S, ce qui correspond à la configuration de la L-proline.

Par "sels d'addition", on entend les sels d'addition d'acide obtenus par réaction d'un composé de formule I avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, benzènesulfonique, maléique, fumarique, oxalique, citrique, lactique et trifluoroacétique.

Le procédé de préparation des composés de formule I, que l'on préconise selon l'invention comprend, selon une première variante A, les étapes consistant à :
(1°) faire réagir un acide de formule : 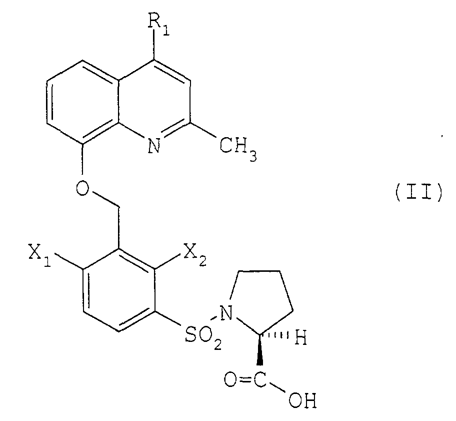
   dans laquelle :
      R₁ représente un atome d'hydrogène ou un groupe alkyle en C₁-C₃ à chaîne hydrocarbonée linéaire ou ramifiée,
      X₁ et X₂ représentent le même halogène,
      avec une amine de formule : 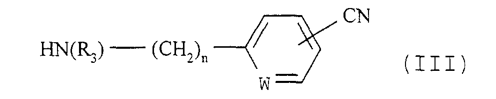
   dans laquelle R₃ représente un atome d'hydrogène ou un groupe méthyle, n représente 0, 1, 2 ou 3, et W représente CH,
   dans un solvant tel que par exemple du dichlorométhane et en présence d'activateurs couramment utilisés pour créer des liaisons de type peptidique tels que par exemple le chlorhydrate de 1-(3-diméthylaminopropyl)-3-éthyl-carbodiimide (EDCI) et le 1-hydroxy-7-aza-benzotriazole (HOAT), à une température proche de la température ambiante (i.e. une température comprise entre environ 0 et environ 40°C, et mieux une température comprise entre 10 et 35°C), pendant 2 à 50 heures, pour obtenir un composé de formule : 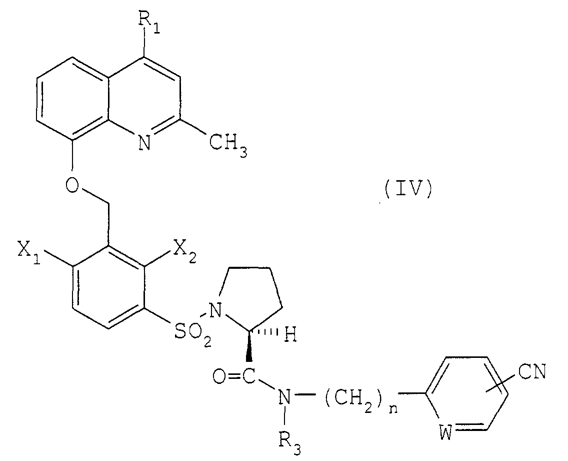
   dans laquelle R₁, R₃, X₁, X₂, n et W conservent la même signification que ci-dessus ;
(2°) faire réagir le composé de formule IV, ainsi obtenu, avec le sulfure d'hydrogène en excès dans un solvant anhydre du type de la pyridine, en présence de triéthylamine, à une température comprise entre 0 et 40° C, pendant 2 à 40 heures pour obtenir un composé de formule : 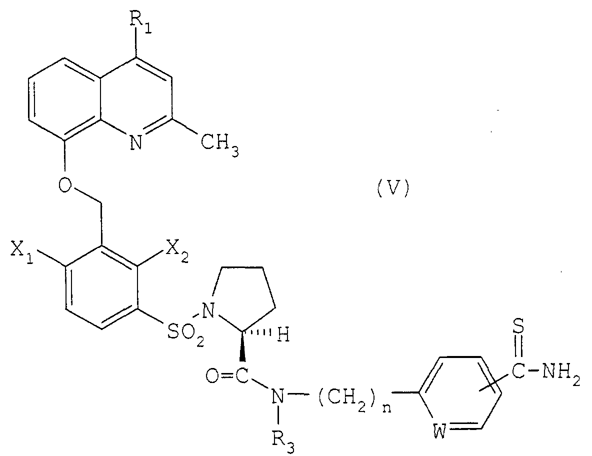 dans laquelle R₁, R₃, X₁, X₂, n et W conservent la même signification que ci-dessus ;
(3°) faire réagir le composé de formule V, ainsi obtenu, avec un agent de méthylation en excès, de préférence l'iodure de méthyle, dans un solvant tel que par exemple l'acétone, à une température proche de la température d'ébullition du milieu réactionnel, éventuellement sous une pression supérieure à la pression atmosphérique et pendant 1 à 5 heures, pour obtenir un composé de formule : 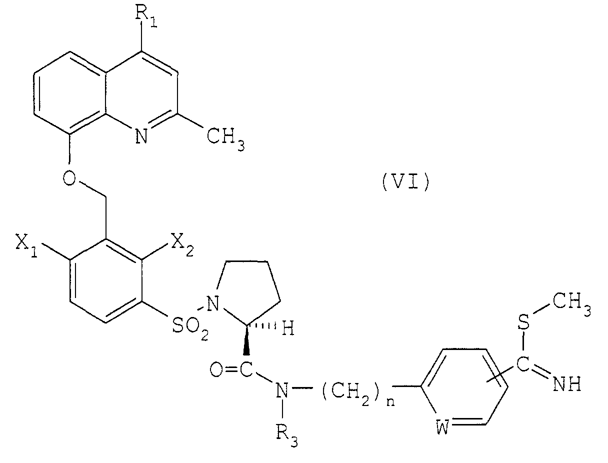 ou l'un de ses sels d'addition,
   où R₁, R₃, X₁, X₂, n et W conservent la même signification que ci-dessus ;
(4°) faire réagir le composé de formule VI, ainsi obtenu, avec un sel d'ammonium, préférentiellement l'acétate d'ammonium, dans un solvant comme par exemple l'éthanol, à une température comprise entre la température ambiante et 100° C, pendant 1 à 10 heures, pour obtenir un composé de formule I : 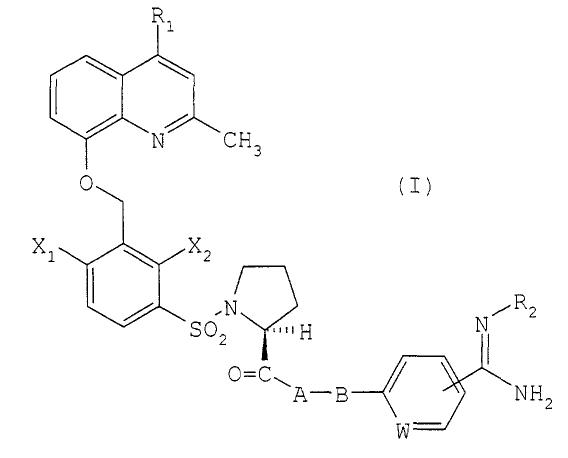 dans laquelle :
   - R₁: représente un atome d'hydrogène ou un groupe alkyle en C₁-C₃ à chaîne hydrocarbonée linéaire ou ramifiée,
   - X₁ et X₂:: représentent le même atome d'halogène,
   - A: représente un groupe -N(R₃)-(CH₂)ₙ-,
   - B: représente une liaison simple,
   - W: représente CH,
   - R₂: représente un atome d'hydrogène,
   - R₃: représente H ou CH₃,
   - n: représente 0, 1, 2 ou 3 ; et
(5°) si nécessaire, faire réagir le composé de formule I, ainsi obtenu, sous forme de base libre avec un acide minéral ou organique pour obtenir le sel d'addition d'acide correspondant ; ou,
   selon une seconde variante B, les étapes consistant à :
   (1°) faire réagir un composé de formule : 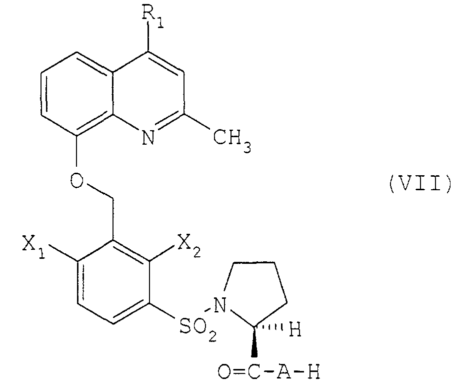 dans laquelle :
      - R₁: représente un atome d'hydrogène ou un groupe alkyle en C₁-C₃ à chaîne hydrocarbonée linéaire ou ramifiée.
      - X₁ et X₂: représentent le même halogène,
      - A: représente un groupe, 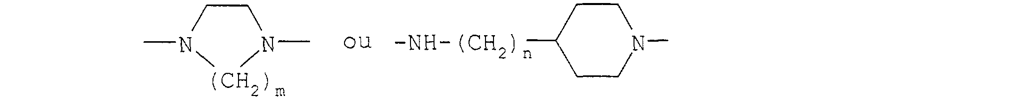
      où n représente 0, 1, 2 ou 3, et m représente 2,
      avec un composé de formule VIII : 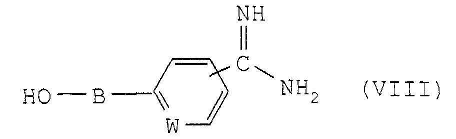
      dans laquelle B représente -CO-, -CO-CH₂-, ou -CO-CH₂-O-,
         selon des conditions analogues à celles de l'étape (1°) de la variante A ci-dessus, pour obtenir un composé de formule : 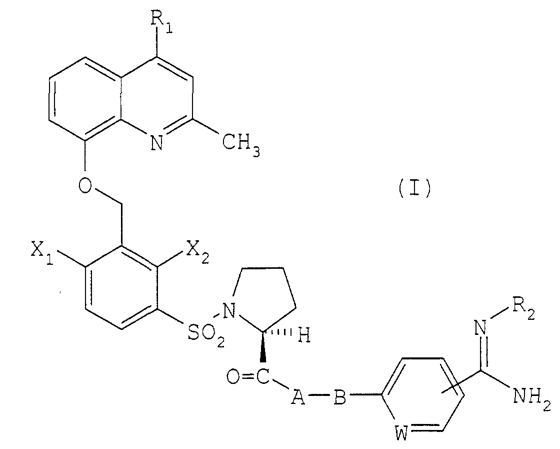
      dans laquelle R₁, X₁ et X₂ conservent la même signification que ci-dessus,
         - A: représente un groupe 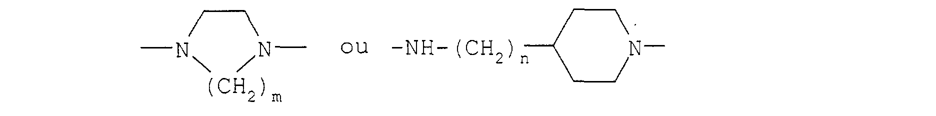
         - B: représente un groupe -CO-, -CO-CH₂- ou -CO-CH₂-O-,
         - m: représente 2,
         - n: représente 0, 1, 2 ou 3,
         - W: représente CH, et
         - R₂: représente un atome d'hydrogène ; et,
   (2°) si nécessaire, faire réagir le composé de formule I, ainsi obtenu, avec un acide pour obtenir le sel d'addition d'acide correspondant ;
      selon une troisième variante C, les étapes consistant à :
      (1°) faire réagir un composé de formule : 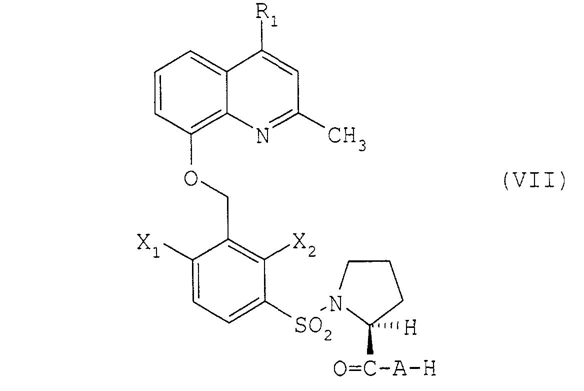 dans laquelle :
         - R₁: représente un atome d'hydrogène ou un groupe alkyle en C₁-C₃, à chaîne hydrocarbonée linéaire ou ramifiée,
         - X₁ et X₂: représentent le même halogène,
         - A: représente un groupe, 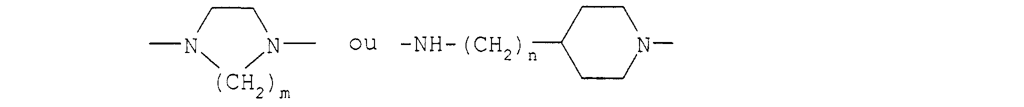
         où m représente 2, et n représente 0, 1, 2 ou 3,
         avec un composé de formule : 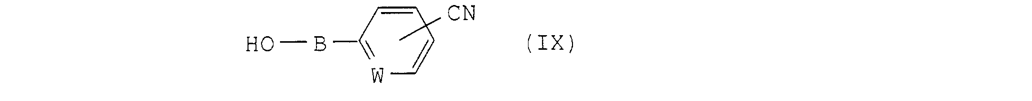 dans laquelle B représente -CO-, -CO-CH₂-, ou -CO-CH₂-O-, et
         W représente CH,
         selon des conditions analogues à celles précédemment décrites pour réaliser l'étape 1° de la variante A et obtenir un composé de formule : 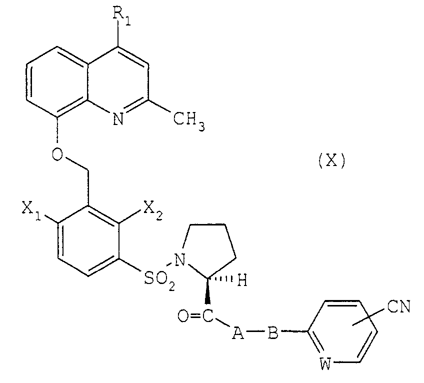 dans laquelle R₁, X₁, X₂, A, B et W conservent la même signification que dans les composés de départ ;
      (2°) faire réagir le composé de formule X, ainsi obtenu, avec de l'hydroxylamine, dans un solvant comme par exemple le DMSO, à température ambiante et pendant 1 à 12 heures, pour obtenir le composé de formule : 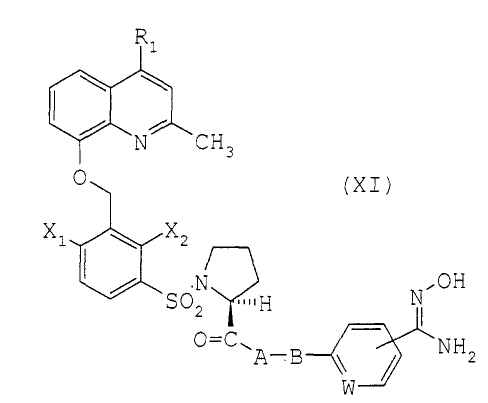 dans laquelle R₁, X₁, X₂, A, B et W conservent la même signification que dans le composés de départ ;
      (3°) faire réagir le composé de formule XI, ainsi obtenu, avec de l'anhydride acétique, de préférence dans un solvant tel que par exemple le dichlorométhane, à température ambiante pendant 1 à 8 heures, pour obtenir le composé de formule : 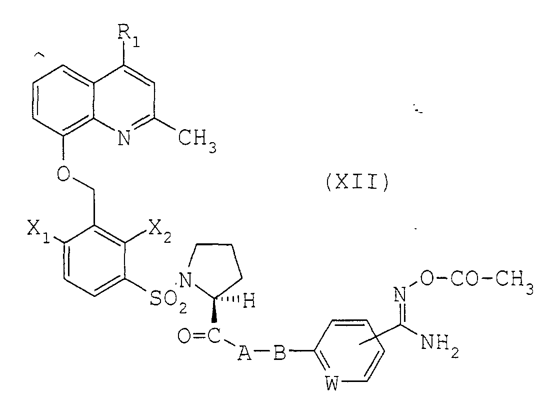 dans laquelle R₁, X₁, X₂, A, B et W conservent la même signification que dans le composés de départ ;
      (4°) effectuer une réduction du composé de formule XII, ainsi obtenu, par hydrogénation catalytique en présence d'un catalyseur tel que par exemple le catalyseur de Lindlar, dans un solvant tel que par exemple le méthanol, sous une pression d'hydrogène d'environ 10⁵ à 10⁶ pascals et à température ambiante pour obtenir le composé de formule : 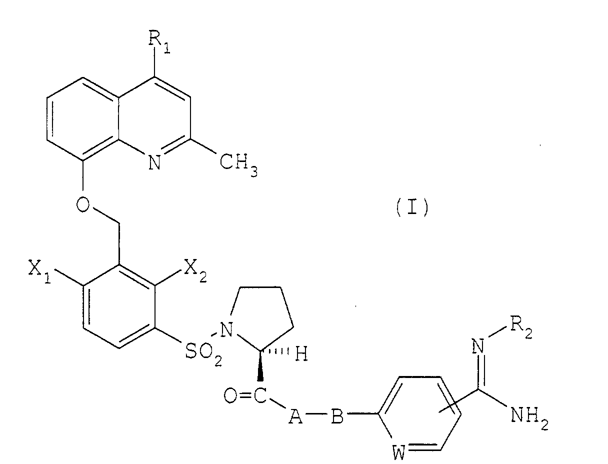 dans laquelle R₁, X₁, X₂, A, B et W conservent la même signification que dans les composés de départ et R₂ représente un atome d'hydrogène ; et,
      (5°) si nécessaire obtenir le sel d'addition du composé de formule I, ainsi obtenu, par réaction avec un acide approprié.

L'invention sera mieux comprise à la lecture des exemples de préparation qui suivent et des résultats d'essais pharmacologiques obtenus avec des composés selon l'invention. Dans le cas de composés présentant dans leur structure un carbone asymétrique, l'absence d'indication particulière, ou la mention (R,S) signifie qu'il s'agit de composés racémiques ; dans le cas de composés présentant une chiralité, celle-ci est indiquée à la suite immédiate de l'indexation du substituant porté par ledit carbone asymétrique ; on utilise alors les signes (R) ou (S), conformément aux règles de Cahn, Ingold et Prelog. La nomenclature utilisée dans les exemples est celle préconisée par les Chemical Abstracts : ainsi certains dérivés de la L-proline peuvent devenir, après réaction de la fonction acide avec une amine, des dérivés de 2-(S)-pyrrolidinecarboxamide.

Dans la partie expérimentale, les "préparations" sont relatives aux composés intermédiaires et les "exemples" sont relatifs aux produits selon l'invention.

Les points de fusion (F) indiqués ci-après sont en général mesurés à l'aide d'un banc Koffler et ne sont pas corrigés, il s'agit alors de points de fusion instantanée.

Les caractéristiques spectrales des signaux de résonance magnétique nucléaire (RMN) sont données pour le proton (¹H) ou pour l'isotope 13 du carbone (¹³C) : on indique le déplacement chimique par rapport au signal du tétraméthylsilane et, entre parenthèses, la forme du signal (s pour singulet, d pour doublet, t pour triplet, q pour quadruplet, m pour multiplet, sl pour signal large) et le nombre de protons concernés par le signal. A titre indicatif, les spectres RMN¹H ont été réalisés à 300 MHz.

### PREPARATION I

### 1-[[3-[(2,4-Diméthylquinolin-8-yl)oxyméthyl]-2,4-dichlorophényl]sulfonyl]-N-[(3-cyanophényl)méthyl]-2(S)-pyrrolidinecarboxamide

On prépare une solution de 4 g (7,85.10⁻³ mole) de N-[[3-[(2,4-diméthylquinolin-8-yl)oxyméthyl]-2,4-dichlorophényl]sulfonyl]-L-proline dans 40 ml de dichlorométhane et on ajoute 1,5 g (7,85.10⁻³ mole) de chlorhydrate de 1-(3-diméthylaminopropyl)-3-éthyl-carbodiimide (EDCI), 1,07 g (7,85.10⁻³ mole) de 1-hydroxy-7-aza-benzotriazole (HOAT), puis 1,14 g (8,63.10⁻³ mole) de 3-(aminométhyl)-benzonitrile. Le mélange réactionnel est agité à température ambiante (15-25°C) pendant 2 heures. On ajoute alors 10 ml d'une solution 1N d'hydroxyde de sodium puis on décante la phase organique. La phase aqueuse est extraite avec du dichlorométhane puis les phases organiques rassemblées sont lavées à l'eau jusqu'à neutralité, séchées sur sulfate de sodium puis concentrées sous pression réduite. Le produit brut est purifié par chromatographie sur gel de silice avec comme éluant un mélange dichlorométhane/méthanol (98/2 ; v/v). On obtient ainsi 2,6 g du produit attendu sous forme d'un solide blanc (Rendement = 55 %).
F = 94-98°C
[α]_{D}²³ = -51° (c = 0,32 ; CHCl₃)

### PREPARATION II

### 1-[ [3-[(2,4-Diméthylquinolin-8-yl)oxyméthyl] -2,4-dichlorophényl] sulfonyl]-N-[3-(aminothioxométhyl)phénylméthyl]-2(S)-pyrrolidinecarboxamide

On prépare une solution de 2,1 g (3,36.10⁻³ mole) du composé obtenu selon la préparation I, dans 30 ml de pyridine et 3 ml de triéthylamine et on introduit dans cette solution du sulfure d'hydrogène par barbottage pendant 0,5 h à température ambiante, puis on laisse réagir pendant 24 heures à température ambiante. On ajoute ensuite de l'eau puis on extrait le mélange par du dichlorométhane. La phase organique est lavée à l'eau, séchée sur sulfate de sodium et concentrée sous pression réduite. Le produit brut obtenu est purifié par chromatographie sur gel de silice avec comme éluant un mélange dichlorométhane/acétate d'éthyle (1/1 ; v/v). On obtient ainsi 0,8 g du produit attendu sous forme d'un solide jaune (Rendement = 35 %).
F = 116-118°C
[α]_{D}²⁸ = -51° (c = 0,3 ; CHCl₃)

### PREPARATION III

### 1-[[3-[(2,4-Diméthylquinolin-8-yl)oxyméthyl]-2,4-dichlorophényl]sulfonyl]-N-[3-[(imino)(méthylthio)méthyl]phénylméthyl]-2(S)-pyrrolidinecarboxamide, iodhydrate

On prépare une solution de 0,6 g (0,9.10⁻³ mole) du composé obtenu selon la préparation II dans 20 ml d'acétone et on ajoute 1,94 g (13,6.10⁻³ mole) d'iodure de méthyle. On chauffe le mélange réactionnel à doux reflux pendant 2 heures puis on concentre sous pression réduite. On obtient 0,72 g du produit attendu sous forme d'un solide jaune (Rendement = 100 %).
F = 146-148°C
[α]_{D}²⁸ = - 10° (c = 0,3 ; CH₃OH)

### Exemple 1

### 1-[[3-[(2,4-Diméthylquinolin-8-yl)oxyméthyl]-2,4-dichlorophényl]sulfonyl]-N-[3-(aminoiminométhyl)phénylméthyl]-2(S)-pyrrolidinecarboxamide

On porte à reflux pendant 2 heures une solution de 0,72 g (0,9.10⁻³ mole) du composé obtenu selon la préparation III et de 0,21 g (2,7.10⁻³ mole) d'acétate d'ammonium dans 20 ml d'éthanol. Après élimination du solvant sous pression réduite, on ajoute 10 ml d'une solution aqueuse N d'hydroxyde de sodium et on extrait avec du dichlorométhane. Les phases organiques rassemblées sont lavées à l'eau, séchées sur sulfate de sodium puis concentrées sous pression réduite. Le produit obtenu est purifié par chromatographie sur gel de silice greffé NH₂ (Lichroprep® -NH₂ commercialisé par Merck) avec comme éluant un mélange dichlorométhane/méthanol (98/2 ; v/v). On obtient ainsi 0,19 g du produit attendu sous forme d'un solide blanc (Rendement = 30 %).

### Exemple 2

### 1-[[3-[(2,4-Diméthylquinolin-8-yl)oxyméthyl]-2,4-dichlorophényl]sulfonyl]-N-[3-(aminoiminométhyl)phénylméthyl]-2(S)-pyrrolidinecarboxamide, dichlorhydrate

On dissout 0,19 g (0,28.10⁻³ mole) du composé obtenu selon l'exemple 1 dans 1 ml d'acide chlorhydrique 1N puis on lyophilise cette solution. On obtient ainsi 0,2 g du produit attendu sous forme d'un solide jaune pâle.
F = 194-196°C
[α]_{D}²³ = 22° (c = 0,34 ; CH₃OH)

### PREPARATION IV

### 1-[[3-[(2,4-Diméthylquinolin-8-yl)oxyméthyl]-2,4-dichlorophényl]sulfonyl]-N-méthyl-N-[4-cyano-phénylméthyl]-2(S)-pyrrolidinecarboxamide

En opérant de façon analogue à la préparation I, au départ de 4-[(N-méthyl)aminométhyl]benzonitrile, on obtient le produit attendu sous forme d'un solide jaune (Rendement = 80 %).
F = 64°C
[α]_{D}²³ = -39° (c = 0,55 ; CH₃OH)

### PREPARATION V

### 1-[[3-[(2,4-Diméthylquinolin-8-yl)oxyméthyl]-2,4-dichlorophényl]sulfonyl]-N-méthyl-N-[4-(aminothioxométhyl)phénylméthyl]-2(S)-pyrrolidinecarboxamide

En opérant de façon analogue à la préparation II, au départ du composé obtenu selon la préparation IV, on obtient le produit attendu (Rendement = 60 %).
F = 50°C
[α]_{D}²³ = - 44° (c = 0,36 ; CH₃OH)

### PREPARATION VI

### 1-[[3-[(2,4-Diméthylquinolin-8-yl)oxyméthyl]-2,4-dichlorophényl]sulfonyl]-N-méthyl-N-[4-[(imino)(méthylthio)méthyl]phénylméthyl]-2(S)-pyrrolidine-carboxamide

En opérant de façon analogue à la préparation III, au départ du composé obtenu selon la préparation V, on obtient le produit attendu (Rendement = 97 %).
F = 53°C
[α]_{D}²³ = - 7° (c = 0,34 ; CH₃OH)

### Exemple 3

### 1-[[3-[(2,4-Diméthylquinolin-8-yl)oxyméthyl]-2,4-dichlorophényl]sulfonyl]-N-méthyl-N-[4-(aminoiminométhyl)phénylméthyl]-2(S)-pyrrolidinecarboxamide

En opérant de façon analogue à l'exemple 1, au départ du composé obtenu selon la préparation VI, on obtient le produit attendu sous forme d'un solide blanc (Rendement = 50 %).
F = 100°C
[α]_{D}²³ = - 42° (c = 0,31 ; CH₃OH)

### Exemple 4

### 1-[[3-[(2,4-Diméthylquinolin-8-yl)oxyméthyl]-2,4-dichlorophényl]sulfonyl]-N-méthyl-N-[4-(aminoiminométhyl)phénylméthyl]-2(S)-pyrrolidinecarboxamide, dichlorhydrate

En opérant de façon analogue à l'exemple 2, au départ du composé obtenu selon l'exemple 3, on obtient le produit attendu sous forme d'un solide jaune pâle (Rendement = 70 %).
F = 205°C
[α]_{D}²³ = 16° (c = 0,30 ; CH₃OH)

### PREPARATION VII

### 1-[[3-[(2,4-Diméthylquinolin-8-yl)oxyméthyl]-2,4-dichlorophényl]sulfonyl]-N-[4-cyano-phénylméthyl]-2(S)-pyrrolidinecarboxamide

En opérant de façon analogue à la préparation I, au départ de 4-(aminométhyl)benzonitrile, on obtient le produit attendu (Rendement = 65 %).
F = 96-98°C
[α]_{D}²⁹ = -56° (c = 0,32 ; CHCl₃)

### PREPARATION VIII

### 1-[[3-[(2,4-Diméthylquinolin-8-yl)oxyméthyl]-2,4-dichlorophényl]sulfonyl]-N-[4-(aminothioxométhyl)phénylméthyl]-2(S)-pyrrolidinecarboxamide

En opérant de façon analogue à la préparation II, au départ du composé obtenu selon la préparation VII, on obtient le produit attendu (Rendement = 35 %).
F = 106-110°C
[α]_{D}²⁹ = + 21° (c = 0,28 ; CHCl₃)

### PREPARATION IX

### 1-[[3-[(2,4-Diméthylquinolin-8-yl)oxyméthyl]-2,4-dichlorophényl]sulfonyl]-N-[4-[(imino)(méthylthio)méthyl]phénylméthyl]-2(S)-pyrrolidinecarboxamide

En opérant de façon analogue à la préparation III, au départ du composé obtenu selon la préparation VIII, on obtient le produit attendu (Rendement = 95 %).
F = 150-152° C
[α]_{D}²³ = - 10° (c = 0,30 ; CH₃OH)

### Exemple 5

### 1-[[3-[(2,4-Diméthylquinolin-8-yl)oxyméthyl]-2,4-dichlorophényl]sulfonyl]-N-[4-(aminoiminométhyl)phénylméthyl]-2(S)-pyrrolidinecarboxamide

En opérant de façon analogue à l'exemple 1, au départ du composé obtenu selon la préparation IX, on obtient le produit attendu sous forme d'un solide blanc (Rendement = 57 %).
F = 115-118°C
[α]_{D}²⁵ = - 42° (c = 0,29 ; CH₃OH)

### Exemple 6

### 1-[[3-[(2,4-Diméthylquinolin-8-yl)oxyméthyl]-2,4-dichlorophényl]sulfonyl]-N-[4-(aminoiminométhyl)phénylméthyl]-2(S)-pyrrolidinecarboxamide, bis(méthane-sulfonate)

On prépare une solution de 160 mg (0,25.10⁻³ mole) du composé obtenu selon l'exemple 5 dans 5 ml d'éthanol puis on ajoute une solution de 50,4 mg (0,525.10⁻³ mole) d'acide méthanesulfonique dans 2 ml d'éthanol. On maintient le milieu réactionnel pendant 0,5 h sous agitation à température ambiante, puis on le verse lentement dans 50 ml d'éther éthylique. Il se forme un précipité que l'on filtre après environ 0,5 h. Le solide est lavé à l'éther puis repris en solution dans 20 ml d'eau distillée. La solution obtenue est lyophilisée et on obtient ainsi 210 mg du produit attendu sous forme d'un solide blanc (Rendement = 86 %).
F = 180°C
[α]_{D}²⁵ = - 25° (c = 0,32 ; CH₃OH)

### PREPARATION X

### 1-[[3-[(2-Méthylquinolin-8-yl)oxyméthyl]-2,4-dichlorophényl]sulfonyl]-N-[4-cyanophénylméthyl]-2(S)-pyrrolidinecarboxamide

En opérant de façon analogue à la préparation VII, au départ de N-[[3-[(2-méthylquinolin-8-yl)oxyméthyl]-2,4-dichlorophényl]sulfonyl]-L-proline, on obtient le produit attendu (Rendement = 76 %).
F = 86-90°C
[α]_{D}³¹ = - 46° (c = 0,31 ; CH₃OH)

### PREPARATION XI

### 1-[[3-[(2-Méthylquinolin-8-yl)oxyméthyl]-2,4-dichlorophényl]sulfonyl]-N-[4-(aminothioxométhyl)phénylméthyl]-2(S)-pyrrolidinecarboxamide

En opérant de façon analogue à la préparation II, au départ du composé obtenu selon la préparation X, on obtient le produit attendu (Rendement = 47 %).
F = 130-132°C
[α]_{D}³¹ = + 24° (c = 0,30 ; CHCl₃)

### PREPARATION XII

### 1-[[3-[(2-Méthylquinolin-8-yl)oxyméthyl]-2,4-dichlorophényl]sulfonyl]-N-[4-[(imino)(méthylthio)méthyl]phénylméthyl]-2(S)-pyrrolidinecarboxamide

En opérant de façon analogue à la préparation III, au départ du composé obtenu selon la préparation XI, on obtient le produit attendu (Rendement = 99 %).
F = 160-162°C
[α]_{D}³¹ = - 20° (c = 0,30 ; CH₃OH)

### Exemple 7

### 1-[[3-[(2-Méthylquinolin-8-yl)oxyméthyl]-2,4-dichlorophényl]sulfonyl]-N-[4-(aminoiminométhyl)phénylméthyl]-2(S)-pyrrolidinecarboxamide

En opérant de façon analogue à l'exemple 1, au départ du composé obtenu selon la préparation XII, on obtient le produit attendu sous forme d'un solide blanc (Rendement = 35 %).

### Exemple 8

### 1-[[3-[(2-Méthylquinolin-8-yl)oxyméthyl]-2,4-dichlorophényl]sulfonyl]-N-[4-(aminoiminométhyl)phénylméthyl]-2(S)-pyrrolidinecarboxamide, dichlorhydrate

En opérant de façon analogue à l'exemple 2, au départ du composé obtenu selon l'exemple 7, on obtient le produit attendu sous forme d'un solide jaune pâle (Rendement = 98 %).
F = 178-180°C
[α]_{D}²² = - 35° (c = 0,35 ; CH₃OH)

### PREPARATION XIII

### 1-[[3-[(2-Méthylquinolin-8-yl)oxyméthyl]-2,4-dichlorophényl]sulfonyl]-N-[3-cyanophényl]-2(S)-pyrrolidinecarboxamide

En opérant de façon analogue à la préparation X, au départ de 3-aminobenzonitrile, on obtient le produit attendu (Rendement = 75 %).
F = 112-114°C
[α]_{D}²¹ = - 53° (c = 0,33 ; CH₃OH)

### PREPARATION XIV

### 1-[[3-[(2-Méthylquinolin-8-yl)oxyméthyl]-2,4-dichlorophényl)sulfonyl]-N-[3-(aminothioxométhyl)phényl]-2(S)-pyrrolidinecarboxamide

En opérant de façon analogue à la préparation II, au départ du composé obtenu selon la préparation XIII, on obtient le produit attendu (Rendement = 50 %).
F = 134-136°C
[α]_{D}²² = - 130° (c = 0,32 ; CHCl₃)

### PREPARATION XV

### 1-[[3-[(2-Méthylquinolin-8-yl)oxyméthyl]-2,4-dichlorophényl]sulfonyl]-N-[3-[(imino)(méthylthio)méthyl]phényl]-2(S)-pyrrolidinecarboxamide

En opérant de façon analogue à la préparation III, au départ du composé obtenu selon la préparation XIV, on obtient le produit attendu (Rendement = 95 %).
F = 160-164°C
[α]_{D}²² = - 42° (c = 0,32 ; CH₃OH)

### Exemple 9

### 1-[[3-[(2-Méthylquinolin-8-yl)oxyméthyl]-2,4-dichlorophényl]sulfonyl]-N-[3-(aminoiminométhyl)phényl]-2(S)-pyrrolidinecarboxamide

En opérant de façon analogue à l'exemple 1, au départ du composé obtenu selon la préparation XV, on obtient le produit attendu sous forme d'un solide blanc (Rendement = 11 %).
RMN¹H (DMSO)
1,90-2,02 (m , 3H) ; 2,27 (m , 1H) ; 2,60 (s , 3H) ; 3,37-3,40 (m , 1H) ; 3,58 (m , 1H) ; 4,57-4,59 (m , 1H) ; 5,52 (m , 2H) ; 6,47 (m large, 3H) ; 7,29-7,56 (m , 7H) ; 7,65-7,67 (d , 1H) ; 7,76-7,78 (d , 1H) ; 7,90 (m , 1H) ; 8,10-8,13 (d , 1H) ; 8,20-8,23 (d , 1H).

### Exemple 10

### 1-[[3-[(2-Méthylquinolin-8-yl)oxyméthyl]-2,4-dichlorophényl]sulfonyl]-N-[3-(aminoiminométhyl)phényl]-2(S)-pyrrolidinecarboxamide, dichlorhydrate

En opérant de façon analogue à l'exemple 2, au départ du composé obtenu selon l'exemple 9, on obtient le produit attendu sous forme d'un solide jaune pâle (Rendement = 99 %).
F = 192-195°C
[α]_{D}²² = - 37° (c = 0,31 ; CH₃OH)

### PREPARATION XVI

### 1-[[3-[(2,4-Diméthylquinolin-8-yl)oxyméthyl]-2,4-dichlorophényl]sulfonyl]-2(S)-[(pipérazin-1-yl)carbonyl]pyrrolidine

Ce composé est obtenu à partir de son sel d'addition avec l'acide trifluoroacétique, par action d'une solution aqueuse d'hydroxyde de sodium, extraction avec de l'acétate d'éthyle et élimination du solvant sous pression réduite.
F = 169°C
[α]_{D}²⁵ = - 2,7° (c = 0,44 ; CHCl₃)

### Exemple 11

### 1-[[3-[(2,4-Diméthylquinolin-8-yl)oxyméthyl]-2,4-dichlorophényl]sulfonyl]-2(S)-[[4-[4-(aminoiminométhyl)benzoyl]pipérazin-1-yl]carbonyl]pyrrolidine

On prépare une suspension de 0,261 g (1,3.10⁻³ mole) du chlorhydrate de l'acide 4-(aminoiminométhyl)benzoïque dans 10 ml de diméthylformamide et on ajoute 0,182 g (0,95.10⁻³ mole) de EDCI et 0,13 g (0,95.10⁻³ mole) de HOBT. On laisse sous agitation à température ambiante pendant 15 minutes puis on ajoute 0,5 g (0,86.10⁻³ mole) du composé obtenu selon la préparation XVI. Le mélange réactionnel est agité pendant 4 heures à température ambiante puis versé sur de l'eau glacée. On ajoute doucement une solution d'hydroxyde de sodium 1N de façon à amener le pH à 8 et on extrait par du dichlorométhane plusieurs fois. Les phases organiques rassemblées sont lavées à l'eau, séchées sur sulfate de sodium et concentrées sous pression réduite. Le produit brut est purifié par chromatographie sur gel de silice greffé NH₂ (Lichroprep® NH₂) avec comme éluant un mélange dichlorométhane/méthanol (95/5 ; v/v). On obtient ainsi 0,4 g du produit attendu sous forme d'un solide blanc (Rendement = 64 %).
F = 139°C
[α]_{D}²² = - 31° (c = 0,33 ; CH₃OH)

### Exemple 12

### 1-[[3-[(2,4-Diméthylquinolin-8-yl)oxyméthyl]-2,4-dichlorophényl]sulfonyl]-2(S)-[[4-[4-(aminoiminométhyl)benzoyl]pipérazin-1-yl]carbonyl]pyrrolidine, dichlorhydrate

On prépare une solution de 0,35 g (0,48.10⁻³ mole) du composé obtenu selon l'exemple 11, dans 8 ml d'acétate d'éthyle et 2 ml d'éthanol. On ajoute 0,5 ml d'une solution saturée de chlorure d'hydrogène dans l'éther éthylique. Les cristaux formés sont filtrés, lavés avec de l'éther éthylique puis remis en solution dans 20 ml d'eau distillée. La lyophilisation de la solution conduit à 0,35 g de produit attendu sous forme d'un solide jaune pâle (Rendement = 90 %).
F = 211°C
[α]_{D}²² = - 11° (c = 0,34 ; CH₃OH)

### Exemple 13

### 1-[[3-[(2,4-Diméthylquinolin-8-yl)oxyméthyl]-2,4-dichlorophényl]sulfonyl]-2(S)-[[4-[2-[4-(aminoiminométhyl)phényl]-1-oxoéthyl]pipérazin-1-yl]carbonyl]pyrrolidine

En opérant de façon analogue à l'exemple 11, au départ du chlorhydrate de l'acide 4-(aminoiminométhyl)-phénylacétique, on obtient le produit attendu sous forme d'un solide beige (Rendement = 74 %).
F = 126°C
[α]_{D}²⁷ = - 32° (c = 0,32 ; CH₃OH)

### Exemple 14

### 1-[[3-[(2,4-Diméthylquinolin-8-yl)oxyméthyl]-2,4-dichlorophényl]sulfonyl]-2(S)-[[4-[2-[4-(aminoiminométhyl)phényl]-1-oxoéthyl]pipérazin-1-yl]carbonyl]pyrrolidine, dichlorhydrate

En opérant de façon analogue à l'exemple 12, au départ du composé obtenu selon l'exemple 13, on obtient le produit attendu sous forme d'un solide amorphe blanc (Rendement = 88 %).
F = 200°C
[α]_{D}²⁷ = + 20° (c = 0,35 ; CH₃OH)

### Exemple 15

### 1-[[3-[(2,4-Diméthylquinolin-8-yl)oxyméthyl]-2,4-dichlorophényl]sulfonyl]-2(S)-[[4-[2-[4-(aminoiminométhyl)phénoxy]-1-oxoéthyl]pipérazin-1-yl]carbonyl]pyrrolidine

En opérant de façon analogue à l'exemple 11, au départ du chlorhydrate de l'acide 4-(aminoiminométhyl)phénoxyacétique, on obtient le produit attendu sous forme d'un solide beige (Rendement = 77 %).
F = 128°C
[α]_{D}²⁷ = - 32° (c = 0,35 ; CH₃OH)

### Exemple 16

### 1-[[3-[(2,4-Diméthylquinolin-8-yl)oxyméthyl]-2,4-dichlorophényl]sulfonyl]-2(S)-[[4-[2-[4-(aminoiminométhyl)phénoxy]-1-oxoéthyl]pipérazin-1-yl]carbonyl]-pyrrolidine, dichlorhydrate

En opérant de façon analogue à l'exemple 12, au départ du composé obtenu selon l'exemple 15, on obtient le produit attendu sous forme d'un solide couleur crème (Rendement = 95 %).
F = 213°C
[α]_{D}²⁷ = + 22° (c = 0,37 ; CH₃OH)

### Exemple 17

### 1-[[3-[(2-Méthylquinolin-8-yl)oxyméthyl] -2,4-dichlorophényl]sulfonyl]-2(S)-[[4-[4-(aminoiminométhyl)benzoyl]pipérazin-1-yl]carbonyl]pyrrolidine

En opérant de façon analogue à l'exemple 11, au départ de 1-[[3-[(2-méthylquinolin-8-yl)oxyméthyl]-2,4-dichlorophényl]sulfonyl]-2(S)-[(piperazin-1-yl)-carbonyl]pyrrolidine, on obtient le produit attendu sous forme d'un solide blanc (Rendement = 52 %).
F = 152°C
[α]_{D}²⁸ = - 37° (c = 0,32 ; CH₃OH)

### Exemple 18

### 1-[[3-[(2-Méthylquinolin-8-yl)oxyméthyl]-2,4-dichlorophényl]sulfonyl]-2(S)-[[4-[4-(aminoiminométhyl)benzoyl]pipérazin-1-yl]carbonyl]pyrrolidine, dichlorhydrate

En opérant de façon analogue à l'exemple 12, au départ du composé obtenu selon la préparation de l'exemple 17, on obtient le produit attendu sous forme d'un solide blanc crème (Rendement = 95 %).
F = 208°C
[α]_{D}²⁸ = + 10° (c = 0,37 ; CH₃OH)

### Exemple 19

### 1-[[3-[(2-Méthylquinolin-8-yl)oxyméthyl]-2,4-dichlorophényl]sulfonyl]-2(S)-[[4-[2-[4-(aminoiminométhyl)phényl]-1-oxoéthyl]pipérazin-1-yl]carbonyl]pyrrolidine

En opérant de façon analogue à l'exemple 17, au départ du chlorhydrate de l'acide 4-(aminoiminométhyl)-phénylacétique, on obtient le produit attendu sous forme d'un solide blanc (Rendement = 86 %).
F = 155°C
[α]_{D}²⁸ = + 0,6° (c = 0,35 ; DMSO)

### Exemple 20

### 1-[[3-[(2-Méthylquinolin-8-yl)oxyméthyl]-2,4-dichlorophényl]sulfonyl]-2(S)-[[4-[2-[4-(aminoiminométhyl)phényl]-1-oxoéthyl]pipérazin-1-yl]carbonyl]pyrrolidine, dichlorhydrate

En opérant de façon analogue à l'exemple 12, au départ du composé obtenu selon l'exemple 19, on obtient le produit attendu sous forme d'un solide amorphe crème (Rendement = 90 %).
F = 188°C
[α]_{D}³⁰ = + 12° (c = 0,36 ; CH₃OH)

### Exemple 21

### 1-[[3-[(2-Méthylquinolin-8-yl)oxyméthyl]-2,4-dichlorophényl]sulfonyl]-2(S)-[[4-[2-[4-(aminoiminométhyl)phénoxy]-1-oxoéthyl]pipérazin-1-yl]carbonyl]-pyrrolidine

En opérant de façon analogue à l'exemple 17, au départ du chlorhydrate de l'acide 4-(aminoiminométhyl)-phénoxyacétique, on obtient le produit attendu sous forme d'un solide blanc (Rendement = 60 %).
F = 130°C
[α]_{D}³⁰ = - 32,5° (c = 0,36 ; DMSO)

### Exemple 22

### 1-[[3-[(2-Méthylquinolin-8-yl)oxyméthyl]-2,4-dichlorophényl]sulfonyl]-2(S)-[[4-[2-[4-(aminoiminométhyl)phénoxy]-1-oxoéthyl]pipérazin-1-yl]carbonyl]pyrrolidine, dichlorhydrate

En opérant de façon analogue à l'exemple 12, au départ du composé obtenu selon l'exemple 21, on obtient le produit attendu sous forme d'un solide beige clair (Rendement = 86 %,)
F = 215°C
[α]_{D}²⁸ = + 11 ° (c = 0,4 ; CH₃OH)

### Exemple 23

### 1-[[3-[(2-Méthylquinolin-8-yl)oxyméthyl]-2,4-dichlorophényl]sulfonyl]-N-[1-[4-(aminoiminométhyl)benzoyl]pipéridin-4-yl]-2(S)-pyrrolidinecarboxamide

On prépare une solution de 0,48g (2,39.10⁻³ mole) d'acide 4-(aminoiminométhyl)benzoïque dans 40 ml de diméthylformamide et on ajoute 0,5 g (2,63.10⁻³ mole) d'EDCI et 0,36 g (2,63.10⁻³ mole) d'HOAT. Après une heure d'agitation de ce mélange à température ambiante, on ajoute 1,5 g (2,3.10⁻³ mole) du dichlorhydrate de 1-[[3-[(2-méthylquinolin-8-yl)oxyméthyl]-2,4-dichlorophényl]sulfonyl]-N-[pipéridin-4-yl]-2(S)-pyrrolidinecarboxamide et 0,5 g (5,5.10⁻³ mole) de N-méthylmorpholine, et on maintient sous agitation pendant 1 heure. Le milieu réactionnel est ensuite concentré sous pression réduite, repris par 200 ml d'une solution d'hydroxyde de sodium 3N et extrait par du dichlorométhane. La phase organique est lavée à l'eau, séchée sur sulfate de sodium et concentrée sous pression réduite. Le produit brut est purifié par chromatographie sur gel de silice greffé NH₂ (Lichroprep® NH₂) avec comme éluant un mélange dichlorométhane/éthanol (95/5 ; v/v). On obtient ainsi 0,33 g du produit attendu sous forme de cristaux blancs (Rendement = 19 %).
RMN¹H (DMSO)
1,36 (m , 2H) ; 1,70 (m , 1H) ; 1,84 (m , 3H) ; 1,99 (m , 1H) ; 2,13 (m , 1H); 2,60 (s , 3 H) ; 3,02 (m , 1H) ; 3,12 (m , 1H) ; 3,34 (m , 2H) ; 3,53 (m , 1H); 3,74 (m , 1H) ; 4,25 (m , 1H) ; 4,34 (m , 1H) ; 5,53 (s , 2H) ; 7,44 (m , 4H) ; 7,56 (d , 2H) ; 7,85 (m , 3H) ; 7,99 (d, 1H) ; 8,10 (d , 1H) ; 8,22 (d, 1H) ; 8,97 (s large , 3H).

### Exemple 24

### 1-[[3-[(2-Méthylquinolin-8-yl)oxyméthyl]-2,4-dichlorophényl]sulfonyl]-N-[1-[4-(aminoiminométhyl)benzoyl]pipéridin-4-yl]-2(S)-pyrrolidinecarboxamide, bis(méthanesulfonate)

En opérant de façon analogue à l'exemple 6, au départ du composé obtenu selon l'exemple 23, on obtient le produit attendu sous forme d'une poudre blanche (Rendement = 66 %).
F = 184-188°C
[α]_{D}²⁵ = - 6,7 ° (c = 0,67 ; CH₃OH)

### PREPARATION XVII

### 1-[[3-[(2,4-Diméthylquinolin-8-yl)oxyméthyl]-2,4-dichlorophényl]sulfonyl]-N-[pipéridin-4-yl]-2(S)-pyrrolidinecarboxamide, dichlorhydrate

Ce composé est obtenu suivant un procédé analogue à la synthèse du chlorhydrate de 1-[[3-[(2-méthylquinolin-8-yl)oxyméthyl]-2,4-dichlorophényl]-sulfonyl]-N-[pipéridin-4-yl]-2(S)-pyrrolidinecarboxamide, décrite précédemment, en remplaçant la 8-hydroxy-2-méthylquinoléine par la 8-hydroxy-2,4-diméthylquinoléine.
F = 184-186°C
[α]_{D}²⁵ = - 14,2° (c = 0,56 ; CH₃OH)

### PREPARATION XVIII

### 1-[[3-[(2,4-Diméthylquinolin-8-yl)oxyméthyl]-2,4-dichlorophényl]sulfonyl]-N-[(pipéridin-4-yl)méthyl]-2(S)-pyrrolidinecarboxamide, dichlorhydrate

Ce composé est obtenu en opérant de façon analogue au procédé de la préparation XVII, mais en remplaçant le dérivé protégé de la 4-amino-pipéridine par un dérivé protégé de la 4-(aminométhyl)pipéridine.
F = 195°C
[α]_{D}²⁴ = - 32,2° (c = 1 ; CH₃OH)

### Exemple 25

### 1-[[3-[(2,4-Diméthylquinolin-8-yl)oxyméthyl]-2,4-dichlorophényl]sulfonyl]-N-[1-4-(aminoiminométhyl)benzoyl]pipéridin-4-yl]-2(S)-pyrrolidinecarboxamide

En opérant de façon analogue à l'exemple 23, au départ du composé obtenu selon la préparation XVII, on obtient le produit attendu (Rendement = 36 %).
RMN¹H (DMSO)
1,30 (m , 2H) ; 1,70 (m , 1H) ; 1,84 (m , 3H) ; 1,98 (m , 1H) ; 2,11 (m, 1H); 2,55 (s , 3 H) ; 2,62 (s , 3H) ; 3,02 (m , 2H) ; 3,40 (m , 2H) ; 3,56 (m , 1H) ; 3,75 (m , 1H) ; 4,33 (m , 2H) ; 5,52 (s , 2H) ; 6,53 (m large , 3H) ; 7,28 (s , 1H) ; 7,37 (d , 3H) ; 7,48 (t , 1H) ; 7,66 (d, 1H) ; 7,81 (m , 3H) ; 7,96 (d , 1H); 8,09 (d, 1H).

### Exemple 26

### 1-[[3-[(2,4-Diméthylquinolin-8-yl)oxyméthyl]-2,4-dichlorophényl]sulfonyl]-N-[1-[4-(aminoiminométhyl)benzoyl]pipéridin-4-yl]-2(S)-pyrrolidinecarboxamide, bis(méthanesulfonate)

En opérant de façon analogue à l'exemple 6, on obtient le produit attendu sous forme de cristaux blancs (Rendement = 78 %).
F = 186-188°C
[α]_{D}²⁵ = - 9° (c = 0,79 ; CH₃OH)

### Exemple 27

### 1-[[3-[(2,4-Diméthylquinolin-8-yl)oxyméthyl]-2,4-dichlorophényl]sulfonyl]-N-[1-[2-[4-(aminoiminométhyl)phénoxy]-1-oxoéthyl]pipéridin-4-yl]-2(S)-pyrrolidinecarboxamide

En opérant de façon analogue au procédé de l'exemple 23, par réaction du produit obtenu selon la préparation XVII avec l'acide 4-(aminoiminométhyl)-phénoxyacétique, on obtient, le produit attendu (Rendement = 47 %).
RMN¹H (DMSO)
1,25 (m , 2H) ; 1,71 (m , 2H) ; 1,84 (m , 2H); 2,12 (m , 2H) ; 2,55 (s , 3H) ; 2,62 (s , 3 H) ; 2,80 (m , 1H) ; 3,13 (m , 2H) ; 3,40 (m , 1H) ; 3,53 (m , 1H); 3,73 (m, 2H) ; 4,10 (m , 1H) ; 4,33 (m , 1H) ; 4,86 (m , 2H) ; 5,53 (s , 2H) ; 6,45 (m large , 3H) ; 6,90 (d , 2H) ; 7,28 (s , 1H) ; 7,38 (d , 1H) ; 7,48 (t , 1H) ; 7,68 (t , 3H) ; 7,81 ( d , 1H) ; 7,96 (d , 1H) ; 8,10 (d, 1H).

### Exemple 28

### 1-[[3-[(2,4-Diméthylquinolin-8-yl)oxyméthyl]-2,4-dichlorophényl]sulfonyl]-N-[1-[2-[4-(aminoiminométhyl)phénoxy]-1-oxoéthyl]pipéridin-4-yl]-2(S)-pyrrolidinecarboxamide, bis(méthanesulfonate)

En opérant de façon analogue au procédé de l'exemple 6, on obtient le produit attendu sous forme de cristaux blancs (Rendement = 72 %).
F = 170°C
[α]_{D}²¹ = - 4,2° (c = 0,93 ; CH₃OH)

### Exemple 29

### 1-[[3-[(2,4-Diméthylquinolin-8-yl)oxyméthyl]-2,4-dichlorophényl]sulfonyl]-N-[[1-[2-[4-(aminoiminométhyl)phénoxy]-1-oxoéthyl]pipéridin-4-yl]méthyl]-2(S)-pyrrolidinecarboxamide

En opérant de façon analogue à l'exemple 27, au départ du composé obtenu selon la préparation XVIII, on obtient le produit attendu sous forme de cristaux blancs (Rendement = 42 %).
RMN¹H (DMSO)
1,10 (m, 2H) ; 1,64 (m , 3H) ; 1,86 (m , 2H) ; 2,05 (m , 1H) ; 2,14 (m , 1H); 2,56 (s , 3 H) ; 2,64 (s , 3H) ; 2,97 (m , 3H) ; 3,39 (m , 2H) ; 3,56 (m , 1H) ; 3,85 (m , 1H) ; 4,29 (m , 1H) ; 4,36 (m , 1H) ; 4,85 (s , 2H) ;.5,55 (s , 2H) ; 6,60 (m large , 3H) ; 6,92 (d , 2H) ; 7,30 (s , 1H) ; 7,40 (d , 1H) ; 7,50 (t , 1H) ; 7,69 (t , 3H) ; 7,84 (d, 1H) ; 8,05 (m , 1H) ; 8,12 (d, 1H).

### Exemple 30

### 1-[[3-[(2,4-Diméthylquinolin-8-yl)oxyméthyl]-2,4-dichlorophényl]sulfonyl]-N-[[1-[2-[4-(aminoiminométhyl)phénoxy]-1-oxoéthyl]pipéridin-4-yl]méthyl]-2(S)-pyrrolidinecarboxamide, bis(méthanesulfonate)

En opérant de façon analogue à l'exemple 6, au départ du composé obtenu selon l'exemple 29, on obtient le produit attendu sous forme de cristaux blancs (Rendement = 70 %).
F = 170°C
[α]_{D}²¹ = - 19° (c = 0,91 ; CH₃OH)

### Exemple 31

### 1-[[3-[(2,4-Diméthylquinolin-8-yl)oxyméthyl]-2,4-dichlorophényl]sulfonyl]-N-[[1-[2-[4-(aminoiminométhyl)phényl]-1-oxoéthyl]pipéridin-4-yl]méthyl]-2(S)-pyrrolidinecarboxamide

En opérant de façon analogue à l'exemple 23, au départ du composé obtenu selon la préparation XVIII et du chlorhydrate de l'acide 4-(aminoiminométhyl)-phénylacétique, on obtient le produit attendu sous forme de cristaux jaune pâle (Rendement = 44 %).
F = 130-132°C
[α]_{D}²⁰ = - 28° (c = 0,90 ; CH₃OH)

### Exemple 32

### 1-[[3-[(2,4-Diméthylquinolin-8-yl)oxyméthyl]-2,4-dichlorophényl]sulfonyl]-N-[[1-[2-[4-(aminoiminométhyl)phényl]-1-oxoéthyl]pipéridin-4-yl]méthyl]-2(S)-pyrrolidinecarboxamide, dichlorhydrate

En opérant de façon analogue à l'exemple 12, au départ du composé obtenu selon l'exemple 31, on obtient le produit attendu sous forme de cristaux blancs (Rendement = 77 %).
F = 210-214°C
[α]_{D}²⁴ = - 17,5° (c = 0,75 ; CH₃OH)

### Exemple 33

### 1-[[3-[(2,4-Diméthylquinolin-8-yl)oxyméthyl]-2,4-dichlorophényl]sulfonyl]-N-[1-[2-[4-(aminoiminométhyl)benzoyl]pipéridin-4-yl]méthyl]-2(S)-pyrrolidinecarboxamide

En opérant de façon analogue à l'exemple 31, au départ du chlorhydrate de l'acide 4-(aminoiminométhyl)benzoïque, on obtient le produit attendu sous forme d'un solide blanc-cassé (Rendement = 59 %).

### Exemple 34

### 1-[[3-[(2,4-Diméthylquinolin-8-yl)oxyméthyl]-2,4-dichlorophényl]sulfonyl]-N-[1-[2-[4-(aminoiminométhyl)benzoyl]pipéridin-4-yl]méthyl]-2(S)-pyrrolidinecarboxamide, dichlorhydrate

En opérant de façon analogue à l'exemple 12, au départ du composé obtenu selon l'exemple 33, on obtient le produit attendu sous forme de cristaux blancs (Rendement = 77 %).
F = 186-190°C
[α]_{D}²⁵ = -19° (c = 1,05 ; CH₃OH)

### Exemple 35

### 1-[[3-[(2-Méthylquinolin-8-yl)oxyméthyl]-2,4-dichlorophényl]sulfonyl]-N-[[1-[4-(aminoiminométhyl)benzoyl]pipéridin-4-yl]méthyl]-2(S)-pyrrolidinecarboxamide, dichlorhydrate

En opérant de façon analogue à l'exemple 23, au départ de chlorhydrate de l'acide 4(aminoiminométhyl)benzoïque et du dichlorhydrate de 1-[[3-[(2-méthylquinolin-8-yl)oxyméthyl]-2,4-dichlorophényl]sulfonyl]-N-[(pipéridin-4-yl)méthyl]-2(S)-pyrrolidinecarboxamide, et après purification au moyen d'une chromatographie en phase inverse sur gel de silice greffé RP18 avec comme éluant le mélange eau/acétonitrile/acide chlorhydrique (63/32/1 ; v/v/v), on obtient le produit attendu sous forme d'un solide poudreux blanc (Rendement = 39 %).
F = 210°C
[α]_{D}²⁶ = -28° (c = 0,98 ; CH₃OH)

### Exemple 36

### 1-[[3-[(2-Méthylquinolin-8-yl)oxyméthyl]-2,4-dichlorophényl]sulfonyl]-N-[[1-[2-[4-(aminoiminométhyl)phényl]-1-oxoéthyl]pipéridin-4-yl]méthyl]-2(S)-pyrrolidinecarboxamide

En opérant de façon analogue à l'exemple 23, au départ du chlorhydrate de l'acide 4-(aminoiminométhyl)benzoïque et du chlorhydrate de 1-[[3-[(2-méthylquinolin-8-yl)oxyméthyl]-2,4-dichlorophényl]sulfonyl]-N-[(pipéridin-4-yl)méthyl]-2(S)-pyrrolidinecarboxamide, on obtient le produit attendu sous forme de cristaux jaunes (Rendement = 36 %).
F = 120-126°C
[α]_{D}²⁶ = - 33° (c = 0,95 ; CH₃OH)

### Exemple 37

### 1-[[3-[(2-Méthylquinolin-8-yl)oxyméthyl]-2,4-dichlorophényl]sulfonyl]-N-[[1-[2-[4-(aminoiminométhyl)phényl]-1-oxoéthyl]pipéridin-4-yl]méthyl]-2(S)-pyrrolidinecarboxamide, dichlorhydrate

En opérant de façon analogue à l'exemple 12, au départ du composé obtenu selon l'exemple 36, on obtient le produit attendu sous forme de cristaux blancs (Rendement = 77 %).
F = 186-190°C
[α]_{D}²⁶ = - 19° (c = 1,05 ; CH₃OH)

### Exemple 38

### 1-[[3-[(2-Méthylquinolin-8-yl)oxyméthyl]-2,4-dichlorophényl]sulfonyl]-N-[[1-[2-[4-(aminoiminométhyl)phénoxy]-1-oxoéthyl]pipéridin-4-yl]méthyl]-2(S)-pyrrolidinecarboxamide

En opérant de façon analogue à l'exemple 31, au départ du chlorhydrate de l'acide 4-(aminoiminométhyl)-phénoxyacétique, on obtient le produit attendu sous forme de cristaux jaunes (Rendement = 73 %).
F = 134-138°C
[α]_{D}²⁵ = - 29° (c = 1,05 ; C₂H₅OH)

### Exemple 39

### 1-[[3-[(2-Méthylquinolin-8-yl)oxyméthyl]-2,4-dichlorophényl]sulfonyl]-N-[[1-[2-[4-(aminoiminométhyl)phénoxy]-1-oxoéthyl]pipéridin-4-yl]méthyl]-2(S)-pyrrolidinecarboxamide, dichlorhydrate

En opérant de façon analogue à l'exemple 12, au départ du composé obtenu à l'exemple 38, on obtient le produit attendu sous forme de cristaux jaune clair (Rendement = 79 %).
F = 197-200°C
[α]_{D}²⁴ = - 22° (c = 0,95 ; CH₃OH)

### Exemple 40

### 1-[[3-[(2,4-Diméthylquinolin-8-yl)oxyméthyl]-2,4-dichlorophényl]sulfonyl]-N-[1-[2-[4-(aminoiminométhyl)phényl]-1-oxoéthyl]pipéridin-4-yl]-2(S)-pyrrolidine carboxamide

En opérant de façon analogue à l'exemple 31, au départ du composé obtenu selon la préparation XVII, on obtient le produit attendu sous forme de cristaux jaune pâle (Rendement = 50 %).

### Exemple 41

### 1-[[3-[(2,4-Diméthylquinolin-8-yl)oxyméthyl]-2,4-dichlorophényl]sulfonyl]-N-[1-[2-[4-(aminoiminométhyl)phényl]-1-oxoéthyl]pipéridin-4-yl]-2(S)-pyrrolidine carboxamide, dichlorhydrate

En opérant de façon analogue à l'exemple 12, au départ du composé obtenu selon l'exemple 40, on obtient le produit attendu sous forme de cristaux blancs (Rendement = 82 %).
F = 205°C
[α]_{D}²¹ = - 7,7° (c = 1,10 ; CH₃OH)

### Exemple 42

### 1-[[3-[(2-Méthylquinolin-8-yl)oxyméthyl]-2,4-dichlorophényl]sulfonyl]-N-[1-[2-[4-(aminoiminométhyl)phényl]-1-oxoéthyl]pipéridin-4-yl]-2(S)-pyrrolidine carboxamide

En opérant de façon analogue à l'exemple 23, au départ du chlorhydrate de l'acide 4-(aminoiminométhyl)phénylacétique, on obtient le produit attendu sous forme de cristaux jaunes (Rendement = 52 %).
F = 114-120°C
[α]_{D}²⁶ = - 29° (c = 0,95 ; CH₃OH)

### Exemple 43

### 1-[[3-[(2-Méthylquinolin-8-yl)oxyméthyl]-2,4-dichlorophényl]sulfonyl]-N-[1-[2-[4-(aminoiminométhyl)phényl]-1-oxoéthyl]pipéridin-4-yl]-2(S)-pyrrolidinecarboxamide, dichlorhydrate

En opérant de façon analogue à l'exemple 12, au départ du composé obtenu selon l'exemple 42, on obtient le produit attendu sous forme de cristaux blancs (Rendement = 77 %).
F = 189-191°C
[α]_{D}²⁶ = - 10° (c = 0,95 ; CH₃OH)

### Exemple 44

### 1-[[3-[(2-Méthylquinolin-8-yl)oxyméthyl]-2,4-dichlorophényl]sulfonyl]-N-[1-[2-[4-(aminoiminométhyl)phénoxy]-1-oxoéthyl]pipéridin-4-yl]-2(S)-pyrrolidinecarboxamide

En opérant de façon analogue au procédé de l'exemple 42, au départ du chlorhydrate de l'acide 4-(aminoiminométhyl)phénoxyacétiqùe, on obtient le produit attendu sous forme de cristaux jaunes (Rendement = 59 %).
F = 134-138°C
[α]_{D}²⁴ = - 27° (c = 0,95 ; CH₃OH)

### Exemple 45

### 1-[[3-[(2-Méthylquinolin-8-yl)oxyméthyl]-2,4-dichlorophényl]sulfonyl]-N-[1-[2-[4-(aminoiminométhyl)phénoxy]-1-oxoéthyl]pipéridin-4-yl]-2(S)-pyrrolidinecarboxamide, dichlorhydrate

En opérant de façon analogue à l'exemple 12, au départ du composé obtenu selon l'exemple 44, on obtient le produit attendu sous forme de cristaux blancs (Rendement = 78 %).
F = 195-200°C
[α]_{D}²⁴ = - 9° (c = 1,00 ; CH₃OH)

### PREPARATION XIX

### 1-[[3-[(2,4-Diméthylquinolin-8-yl)oxyméthyl]-2,4-dichlorophényl]sulfonyl]-2(S)-[[4-(3-cyanobenzoyl)pipérazin-1-yl]carbonyl]pyrrolidine

En opérant de façon analogue à l'exemple 11, au départ d'acide 3-cyanobenzoïque, on obtient le produit attendu sous forme d'un solide poudreux blanc (Rendement = 86 %).
F = 131°C
[α]_{D}²³ = - 13° (c = 0,45 ; CHCl₃)

### Exemple 46 (exemple préparatif)

### 1-[[3-[(2,4-Diméthylquinolin-8-yl)oxyméthyl]-2,4-dichlorophényl]sulfonyl]-2(S)-[[4-[3-[(amino)(hydroxyimino)méthyl]benzoyl]pipérazin-1-yl]carbonyl]pyrrolidine

On prépare une solution de 1,02 g (1,44.10⁻³ mole) du composé obtenu selon la préparation XIX dans 16 ml de diméthylsulfoxyde et on ajoute 0,16 g (2,3.10⁻³ mole) de chlorhydrate d'hydroxylamine, puis 0,48 g (4,76.10⁻³ mole) de triéthylamine. Après 4 heures sous agitation à température ambiante, on ajoute à nouveau les mêmes quantités de chlorhydrate d'hydroxylamine et de triéthylamine et on continue l'agitation pendant 12 heures. On verse ensuite le mélange réactionnel sur 200 ml d'eau. Le précipité obtenu est filtré puis séché sous vide. On obtient ainsi 0,7 g du produit attendu sous forme d'un solide fin blanc (Rendement = 66 %).
F = 160°C
[α]_{D}²³ = - 4° (c = 0,50 ; CHCl₃)

### PREPARATION XX

### 1-[[3-[(2,4-Diméthylquinolin-8-yl)oxyméthyl]-2,4-dichlorophényl]sulfonyl]-2(S)-[[4-[3-[(acétoxyimino)(amino)méthyl]benzoyl]pipérazin-1-yl]carbonyl]pyrrolidine

On ajoute 90 mg (0,88.10⁻³ mole) d'anhydride acétique à une solution de 0,62 g (0,84.10⁻³ mole) du composé obtenu selon l'exemple 46 dans 6 ml de tetrahydrofurane et on agite le mélange réactionnel à température ambiante pendant 30 mn. On ajoute 50 ml de dichlorométhane et on lave cette phase organique avec de l'eau jusqu'à neutralité. La phase organique est séchée sur sulfate de magnésium puis concentrée sous pression réduite. On obtient ainsi 0,65 g du produit attendu sous forme d'un solide blanc.
F = 130°C
[α]_{D}²³ = - 8° (c = 0,25 ; CHCl₃)

### Exemple 47

### 1-[[3-[(2,4-Diméthylquinolin-8-yl)oxyméthyl]-2,4-dichlorophényl]sulfonyl]-2(S)-[[4-[3-(aminoiminométhyl)benzoyl]pipérazin-1-yl]carbonyl]pyrrolidine

On prépare une solution de 0,6 g (0,76.10⁻³ mole) du composé obtenu selon la préparation XX dans 10 ml de méthanol, on ajoute 30 mg de catalyseur de Lindlar (à 5 % de palladium) et on agite ce mélange sous atmosphère d'hydrogène, à pression atmosphérique et à température ambiante pendant 6 heures. Après élimination du catalyseur par filtration, la solution est concentrée sous pression réduite. Le produit brut obtenu est purifié par chromatographie sur gel de silice greffé NH₂ (Lichroprep® NH₂) avec comme éluant le mélange dichlorométhane/méthanol (97/3 ; v/v). On obtient ainsi 0,47 g du produit attendu sous forme d'un solide jaune-pâle (Rendement = 85 %).
F = 158°C
[α]_{D}²² = + 10° (c = 0,50 ; CHCl₃)

### Exemple 48

### 1-[[3-[(2,4-Diméthylquinolin-8-yl)oxyméthyl]-2,4-dichlorophényl]sulfonyl]-2(S)-[[4-[3-(aminoiminométhyl)benzoyl]pipérazin-1-yl]carbonyl]pyrrolidine, dichlorhydrate

On prépare une solution de 380 mg (0,52.10⁻³ mole) du composé obtenu selon l'exemple 47 dans 4 ml de dichlorométhane et on ajoute 1 ml d'une solution saturée de chlorure d'hydrogène dans l'éther éthylique. Après 30 mn d'agitation du mélange on filtre le précipité obtenu et on le lave avec un peu d'éther éthylique. Après séchage, le produit est remis en solution dans 6 ml d'eau, filtré et lyophilisé. On obtient ainsi 389 mg du produit attendu sous forme d'un solide blanc.
F = 210°C
[α]_{D}²⁰ = + 22° (c = 0,65 ; CH₃OH)

### Exemple 49

### 1-[[3-[(2,4-Diméthylquinolin-8-yl)oxyméthyl]-2,4-dichlorophényl]sulfonyl]-N-[3-[4-(aminoiminométhyl)phényl]propyl]-2(S)-pyrrolidinecarboxamide

On prépare une solution de 1 g (1,96.10⁻³ mole) de 1-[[3-[(2,4-diméthylquinolin-8-yl)oxyméthyl]-2,4-dichlorophenyl]sulfonyl]-L-proline dans 10 ml de DMF et on ajoute 0,41 g (2,16.10⁻³ mole) de EDCI et 0,29 g (2,16.10⁻³ mole) de HOAT. Après 30 mn sous agitation à température ambiante, on ajoute au milieu réactionnel une solution de 0,54 g (2,16.10⁻³ mole) de dichlorhydrate de 3-[4-(aminoiminométhyl)phényl]propanamine dans 7 ml de DMF et 0,22 g (2,16.10⁻³ mole) de N-méthylmorpholine. Après 14 heures sous agitation à température ambiante, on verse le mélange réactionnel dans 150 ml d'eau glacée et 10 ml d'une solution 1N d'hydroxyde de sodium. Le produit précipité est filtré et repris en solution dans du dichlorométhane. La solution est lavée à l'eau puis séchée sur sulfate de magnésium et concentrée sous pression réduite. Le produit brut est purifié par chromatographie sur gel de silice greffé NH₂ avec comme éluant le mélange dichlorométhane éthanol (96/4 ;v/v). On obtient ainsi 500 mg du produit attendu sous la forme d'un solide blanc cristallisé (Rendement = 39 %).
F = 130-134°C
[α]_{D}²⁴ = - 42° (c = 0,33 ; CHCl₃)

### Exemple 50

### 1-[[3-[(2,4-Diméthylquinolin-8-yl)oxyméthyl]-2,4-dichlorophényl]sulfonyl]-N-[3-[4-(aminoiminométhyl)phényl]propyl]-2(S)-pyrrolidinecarboxamide, dichlorhydrate

On prépare une solution de 230 mg (0,34.10⁻³ mole) du composé obtenu selon l'exemple 49 dans 5 ml de méthanol et on ajoute 0,5 ml d'une solution de chlorure d'hydrogène 4N dans l'éther éthylique. Après 15 mn sous agitation, on concentre le mélange réactionnel sous pression réduite, on reprend le résidu en solution dans de l'eau et on lyophilise cette solution. On obtient ainsi 250 mg du produit attendu sous forme de cristaux jaune clair (Rendement = 98 %).
F = 188-190°C
[α]_{D}²⁵ = - 37° (c = 0,31 ; CH₃OH)

### Exemple 51

### 1-[[3-[(2,4-Diméthylquinolin-8-yl)oxyméthyl]-2,4-dichlorophényl]sulfonyl]-N-[2-[4-(aminoiminométhyl)phényl]éthyl]-2(S)-pyrrolidinecarboxamide, dichlorhydrate

En opérant de façon analogue aux exemples 49 et 50, au départ de dichlorhydrate de 2-[4-(aminoiminométhyl)phényl]éthanamine, on obtient le produit attendu sous forme de cristaux blancs (Rendement = 30 %).
F = 190-194°C
[α]_{D}²⁴ = - 22° (c = 0,36 ; CH₃OH)

L'activité des produits selon l'invention a été évaluée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement B₁ et B₂. Le récepteur B₂ appartient à la grande famille des récepteurs à sept domaines transmembranaires couplés aux G-protéines. Nous décrivons dans la présente invention des composés se liant au récepteur B₂ et bloquant de ce fait la fixation de la bradykinine.

Le test pharmacologique utilisé est le suivant : des segments d'iléon de cobayes mâles [de souche Dunkin-Hartley (Iffa Credo, l'Arbresle, France)] sont isolés et broyés dans le tampon TES suivant : TES 25mM, 1,10-phénanthroline 1mM (pH 6.8), bacitracine 140 µg/ml, BSA 1g/l. Les membranes sont ensuite isolées par centrifugation (18000 tours par minute ; 20 min ; 4°C). Les études de liaison sont effectuées dans ce tampon TES en utilisant la [³H]-bradykinine (120 pM), 50 µg de protéine membranaire par essai (volume final 500 µl) avec un temps d'équilibre de 90 min à 20°C. On détermine ensuite le taux (en pourcentage) d'inhibition de la fixation de [³H]-bradykinine en présence de l'un des composés selon l'invention à tester à une concentration de 10⁻⁶ M.

Les résultats obtenus (notés "activité") lors de ces essais sont consignés dans le tableau I ci-après en regard des exemples figurant dans la description.

Les composés de la présente invention, qui inhibent la liaison de la [³H] bradykinine au récepteur B₂ de cobaye (voir tableau I), se lient également au récepteur B₂ humain cloné et transfecté de façon stable dans des cellules CHO (Chinese Hamster Ovary Cells). Ainsi dans ce test, certains composés inhibent à la concentration de 10 µM d'au moins 95 % la fixation de la [³H]-bradykinine au récepteur B₂.

Les composés de la présente invention peuvent être utiles dans le traitement des algies, et en particulier dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastrointestinal (par ex. colites, rectites, maladie de Crohn), la pancréatite, certains carcinomes, l'angiooedème héréditaire, la migraine, l'encéphalomyélite, la méningite, les accidents vasculaires cérébraux (notamment ceux provoqués par un choc traumatique cérébral), certains désordres neurologiques, les états inflammatoires vasculaires (par exemple : athérosclérose et artérite des membres inférieurs), les états douloureux (par exemple les céphalgies, les douleurs dentaires, les douleurs menstruelles), les contractions utérines prématurées, la cystite et les brûlures. Les composés selon l'invention peuvent également être utiles pour la potentialisation d'agents antiviraux.

Les composés de la présente invention, qui peuvent être utilisés sous forme de base libre ou de leurs sels d'addition non-toxiques, en association avec un excipient physiologiquement acceptable, sont en général prescrits en thérapeutique humaine à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Ces composés sont également administrables par voie topique, par exemple sous forme de gel ou de pommade.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [³H] ou du soufre [³⁵S]), dans le but d'obtenir un radio-ligand destiné à des études conformationnelles du récepteur B₂ de la bradykinine, ou des tests de fixation à ce type de récepteur, par exemple pour l'évaluation de nouveaux composés susceptibles de présenter une affinité pour le récepteur B₂ de la bradykinine.

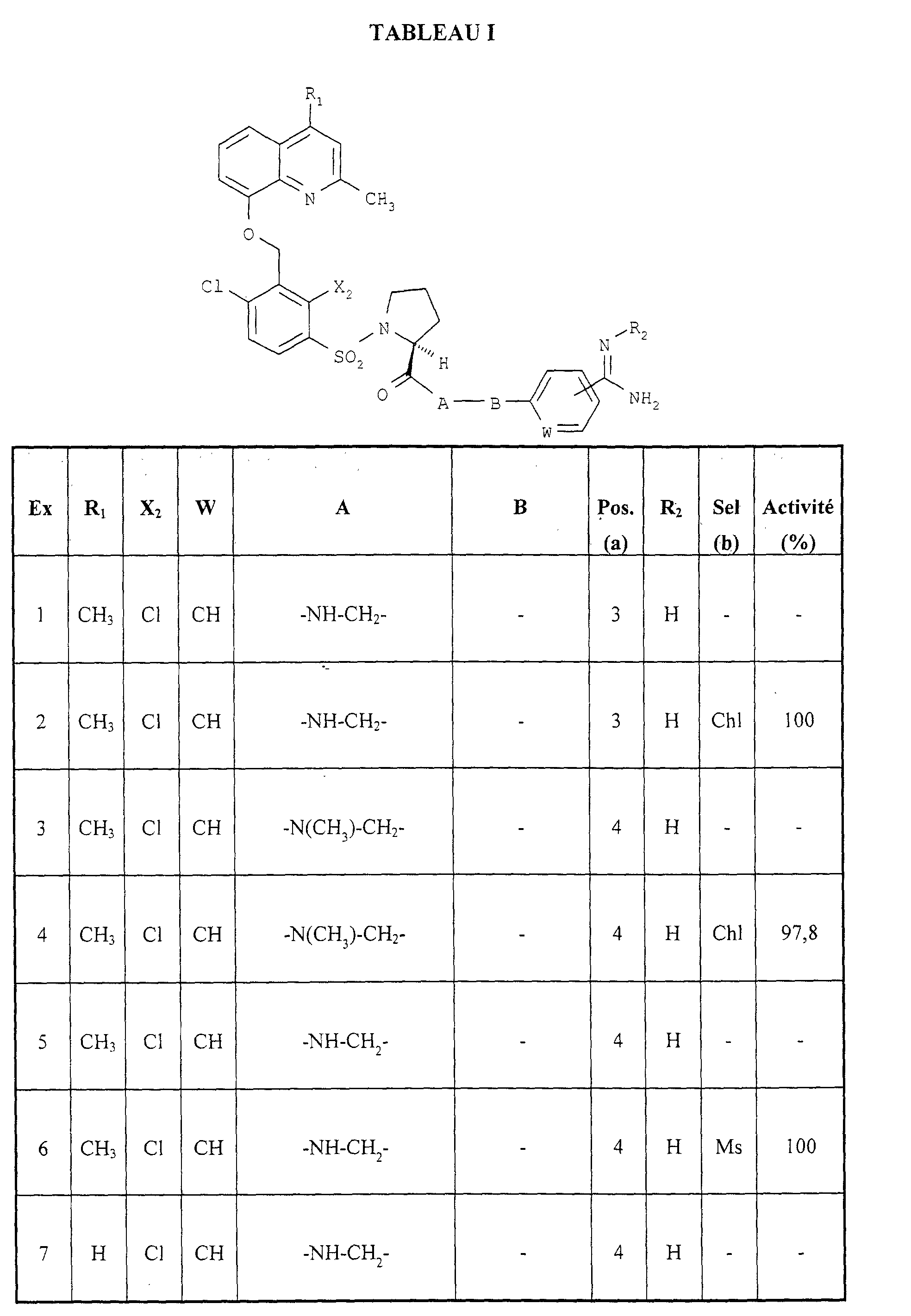

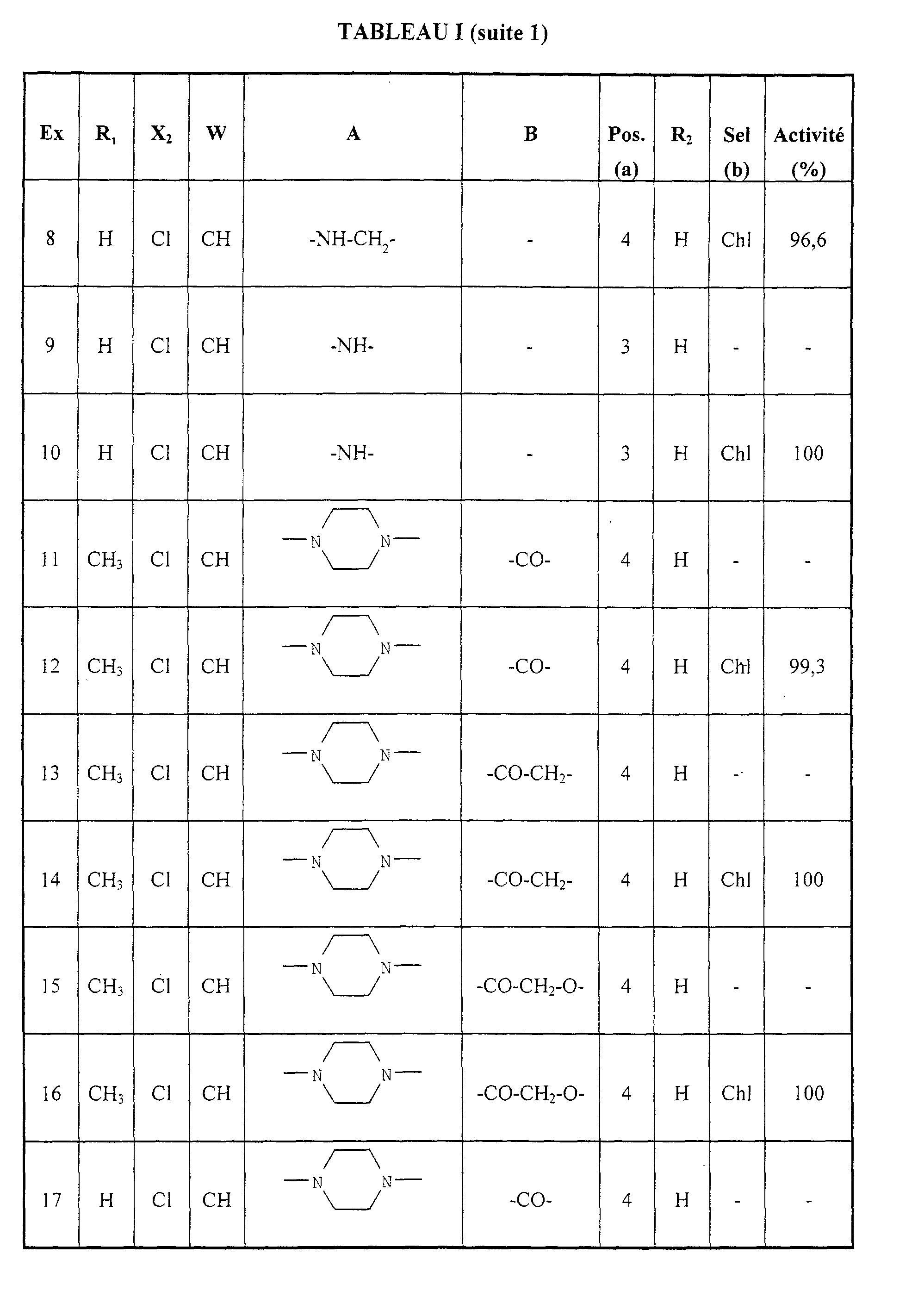

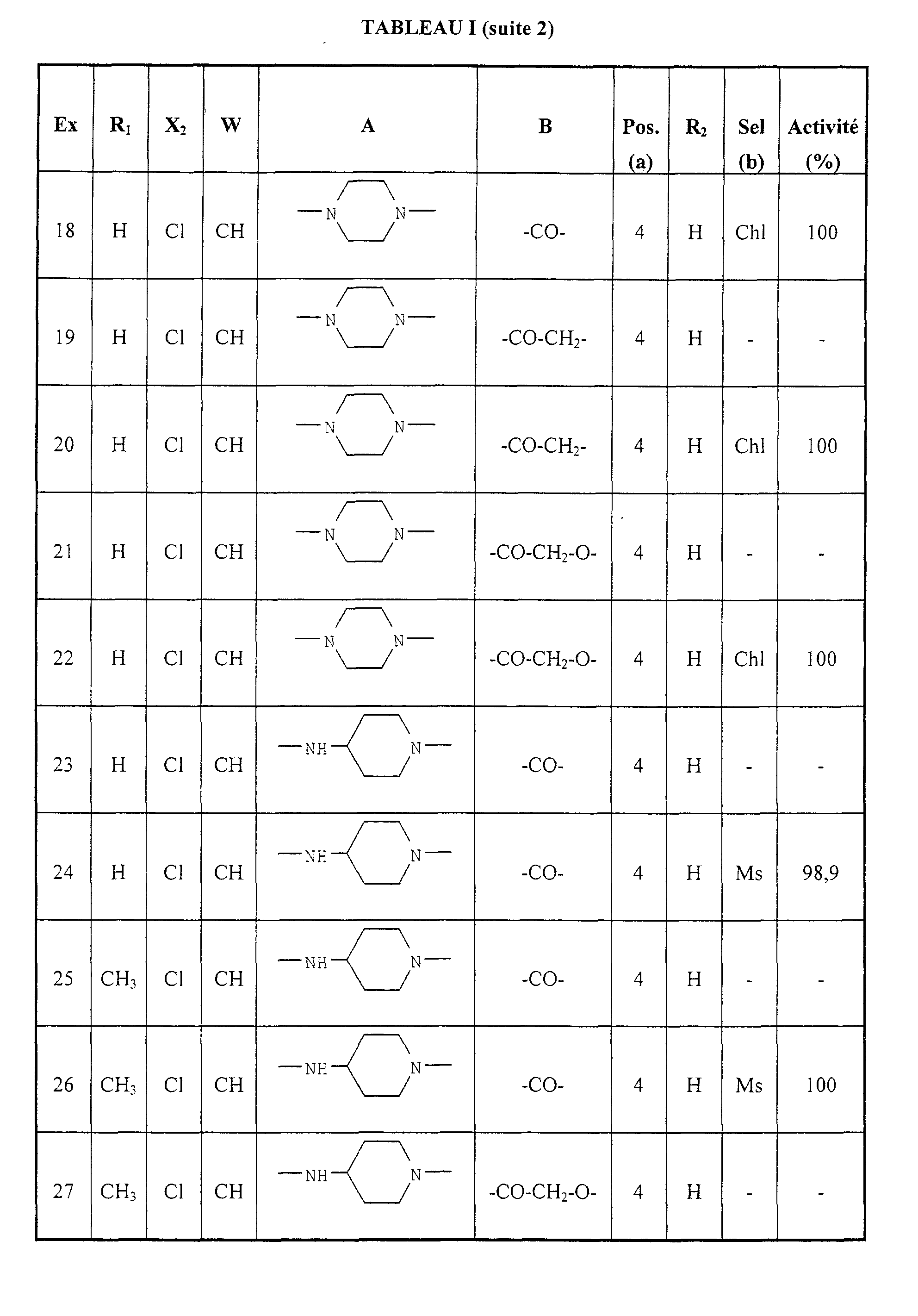

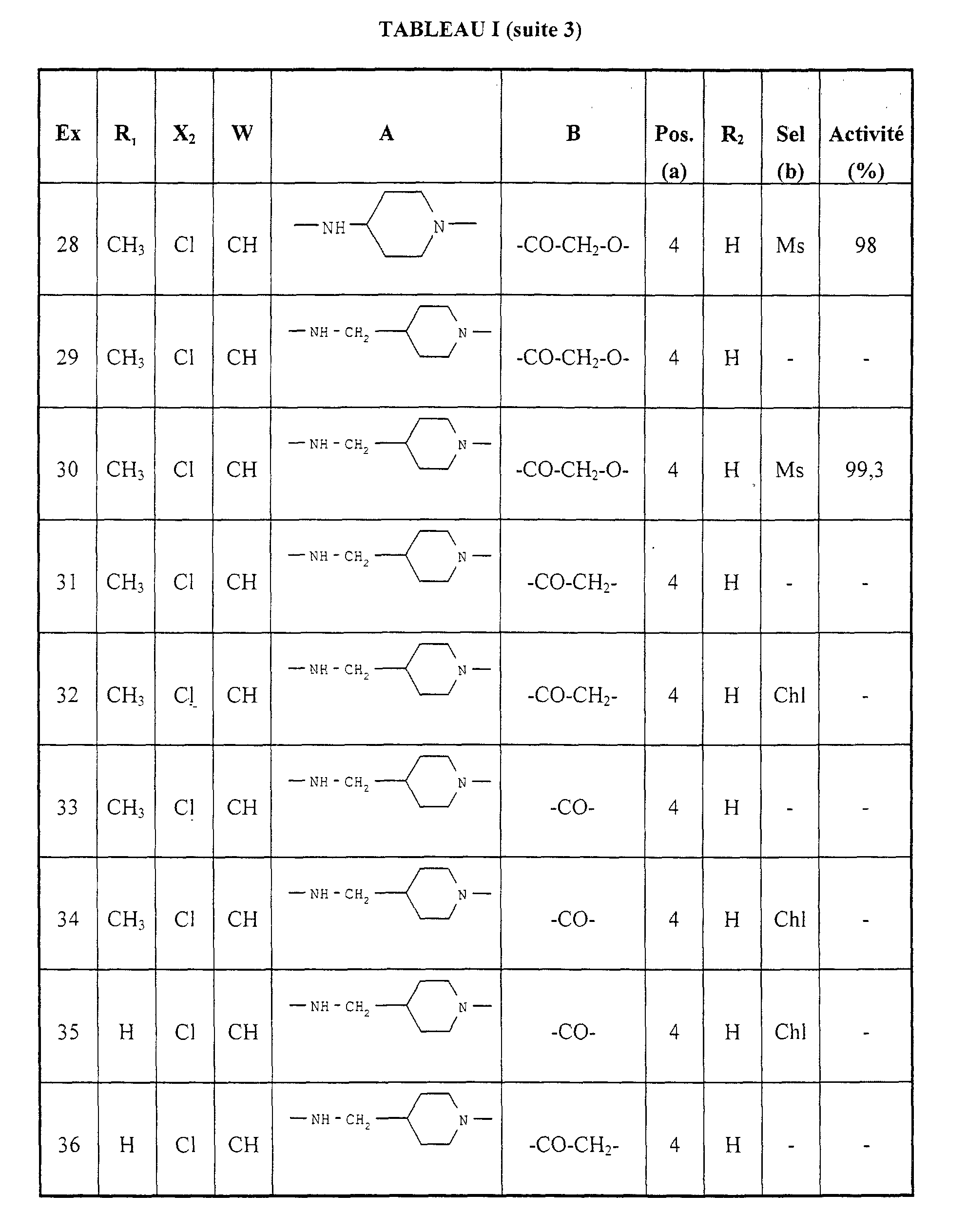

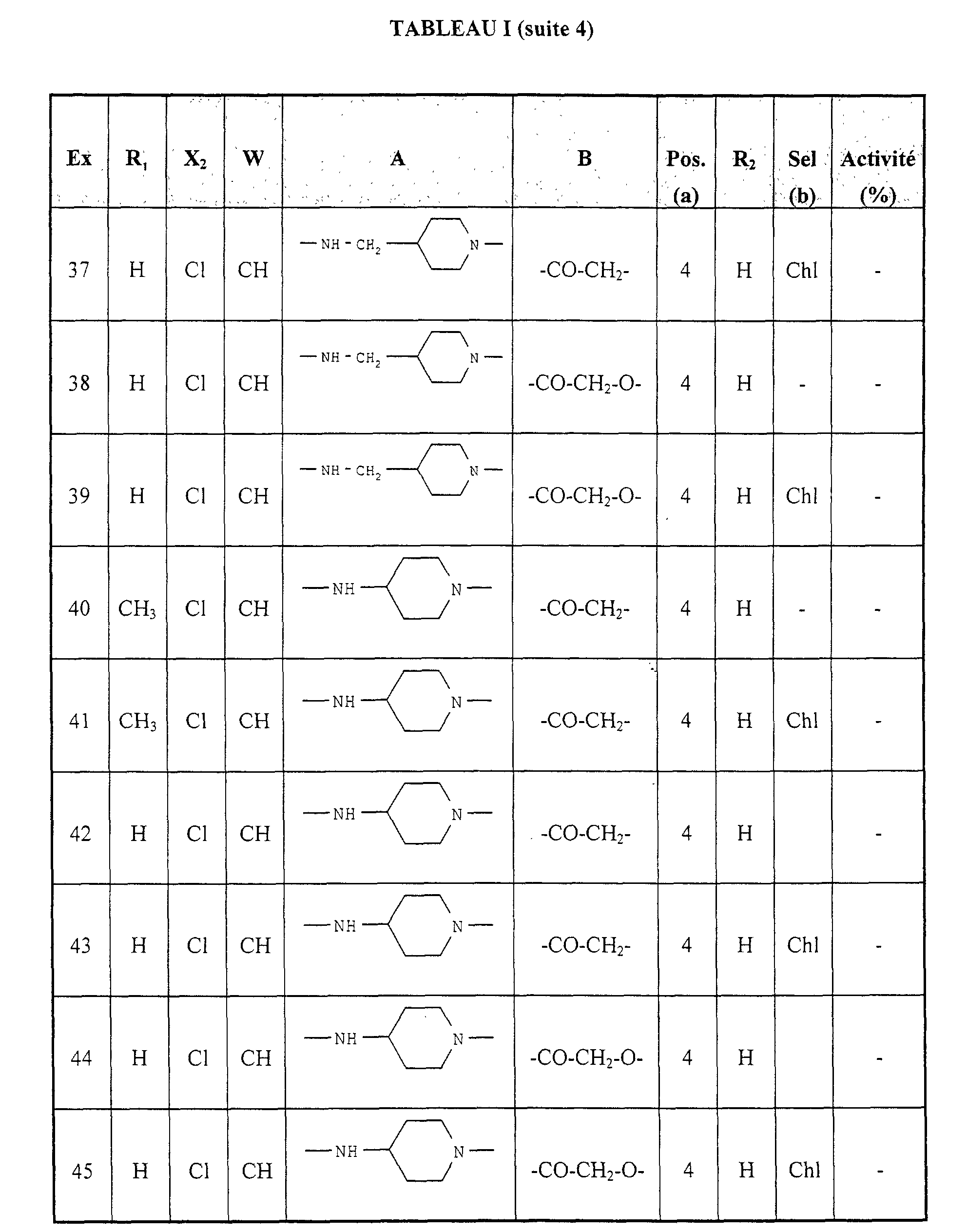

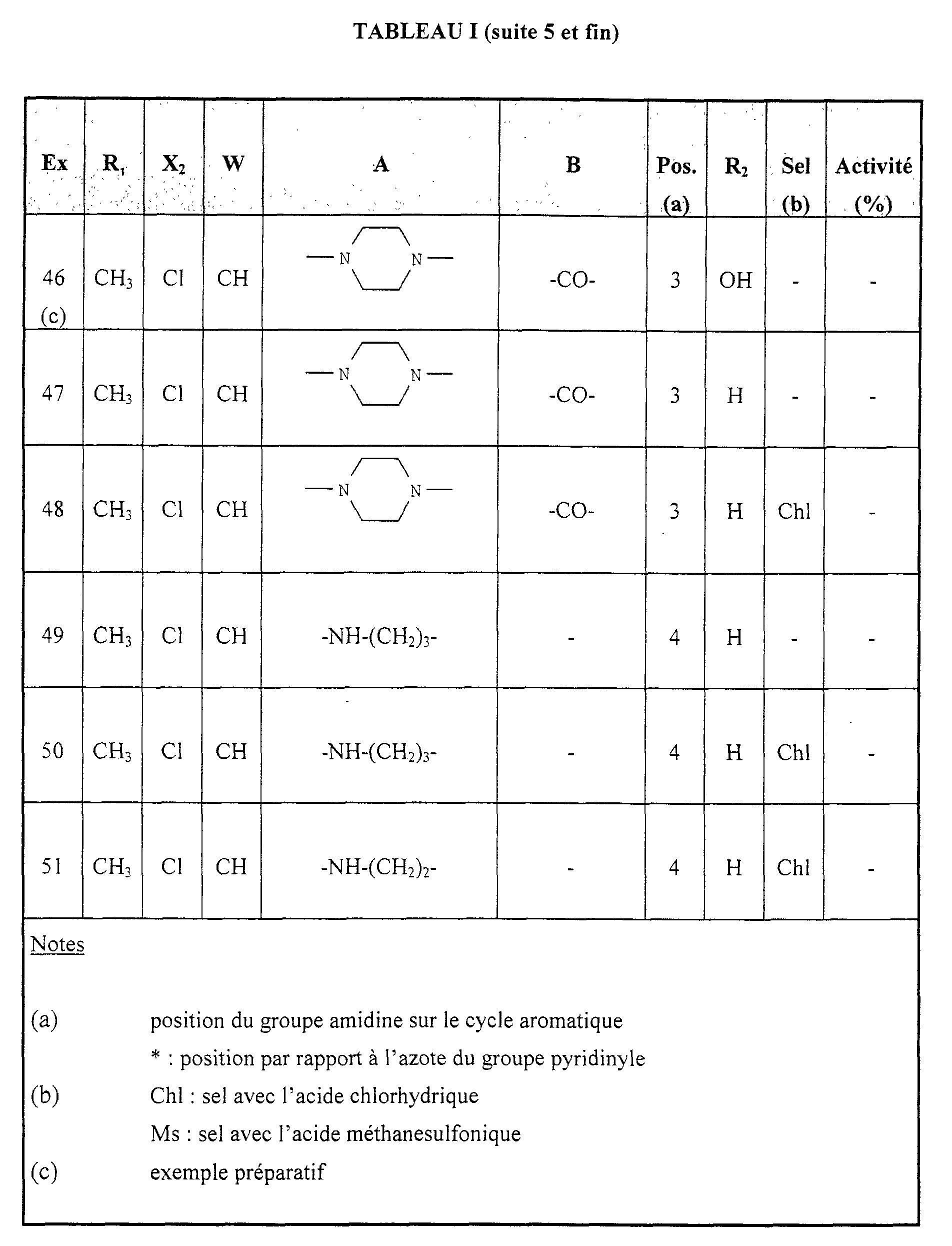

## Revendications

1. Composé dérivé de N-benzènesulfonyl-(L)-proline, **caractérisé en ce qu'**il est choisi parmi l'ensemble constitué par :
(**i**) les composés de formule : 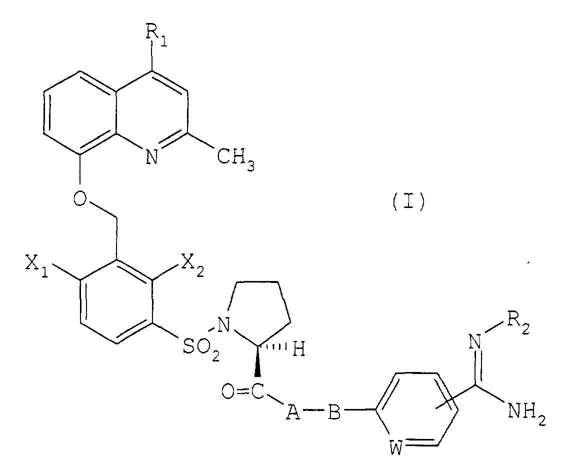 dans laquelle :
X₁ et X₂ représentent le même atome d'halogène,
R₁ représente un atome d'hydrogène ou un groupe alkyle en C₁-C₃ à chaîne hydrocarbonée linéaire ou ramifiée,
R₂ représente un atome d'hydrogène,
A représente un groupe 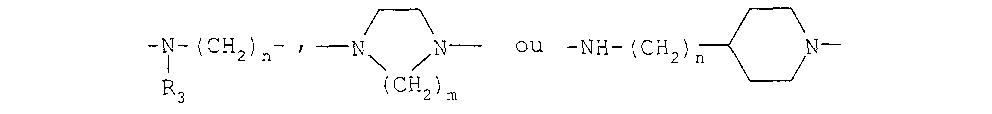
B représente une liaison simple, -CO-, -CO-CH₂-, ou -CO-CH₂-O-,
m représente 2,
n représente 0, 1, 2 ou 3,
R3 représente un atome d'hydrogène ou un groupe méthyle,
W représente CH,
le groupe amidine C(=NR₂)NH₂ étant en position 2, 3 ou 4 sur le noyau aromatique, et,
(**ii**) leurs sels d'addition.

2. Composé selon la revendication 1, **caractérisé en ce que** dans la formule I, X₁ et X₂ sont des atomes de chlore.

3. Procédé de préparation d'un composé de formule I, **caractérisé en ce qu'**il comprend les étapes consistant à :
(1°) faire réagir un acide de formule : 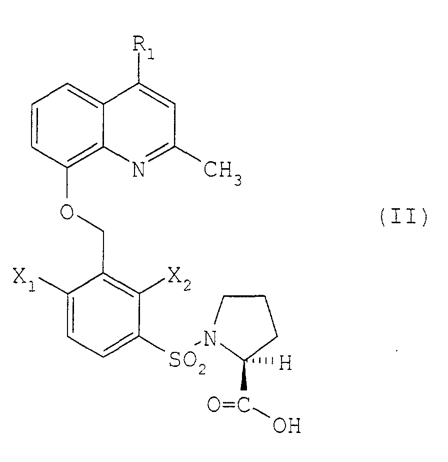 dans laquelle :
R₁ représente un atome d'hydrogène ou un groupe alkyle en C₁-C₃ à chaîne hydrocarbonée linéaire ou ramifiée,
X₁ et X₂ : représentent le même halogène,
avec une amine de formule : 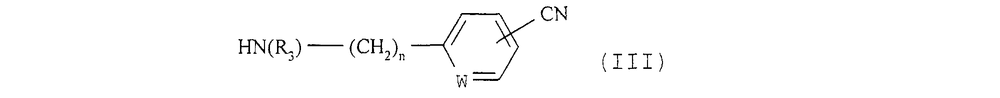 dans laquelle R₃ représente un atome d'hydrogène ou un groupe méthyle,
n représente 0, 1, 2 ou 3, et W représente CH,
dans un solvant et en présence d'activateurs couramment utilisés pour créer des liaisons de type peptidique, à une température proche de la température ambiante, pendant 2 à 50 heures, pour obtenir un composé de formule : 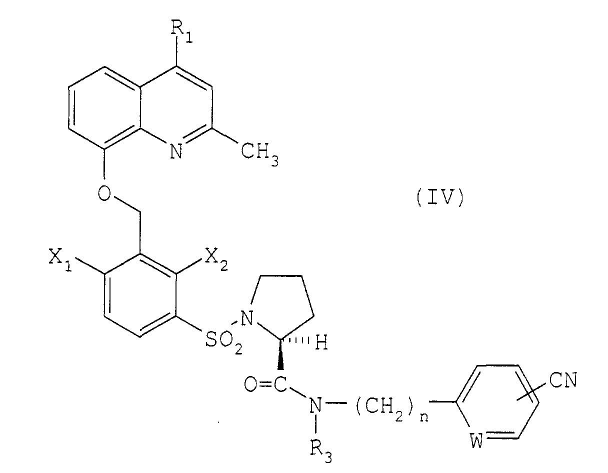 dans laquelle R₁, R₃, X₁, X₂, n et W conservent la même signification que ci-dessus ;
(2°) faire réagir le composé de formule IV, ainsi obtenu, avec le sulfure d'hydrogène en excès dans un solvant anhydre, en présence de triéthylamine, à une température comprise entre 0 et 40°C, pendant 2 à 40 heures pour obtenir un composé de formule : 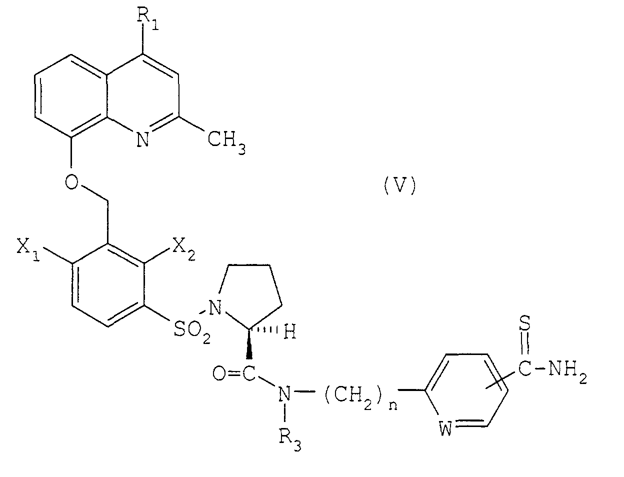 dans laquelle R₁, R₃, X₁, X₂, n et W conservent la même signification que ci-dessus ;
(3°) faire réagir le composé de formule V, ainsi obtenu, avec un excès d'un agent de méthylation, dans un solvant, à une température proche de la température d'ébullition du milieu réactionnel, éventuellement sous une pression supérieure à la pression atmosphérique et pendant 1 à 5 heures, pour obtenir un composé de formule : 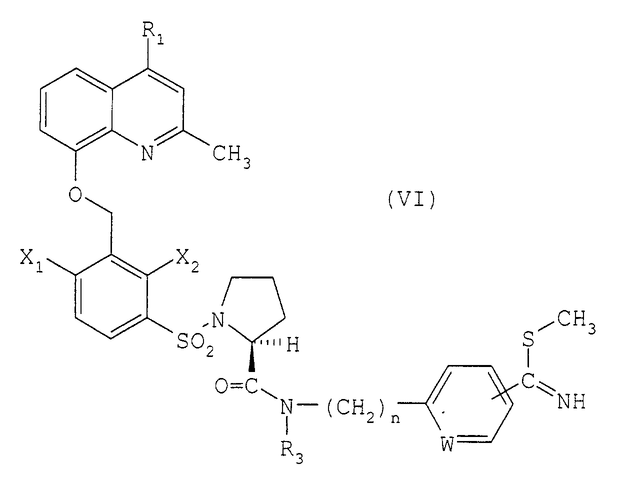 ou l'un de ses sels d'addition,
où R₁, R₃, X₁, X₂, n et W conservent la même signification que ci-dessus ;
(4°) faire réagir le composé de formule VI, ainsi obtenu, avec un sel d'ammonium, dans un solvant, à une température comprise entre la température ambiante et 100°C, pendant 1 à 10 heures, pour obtenir un composé de formule 1 : 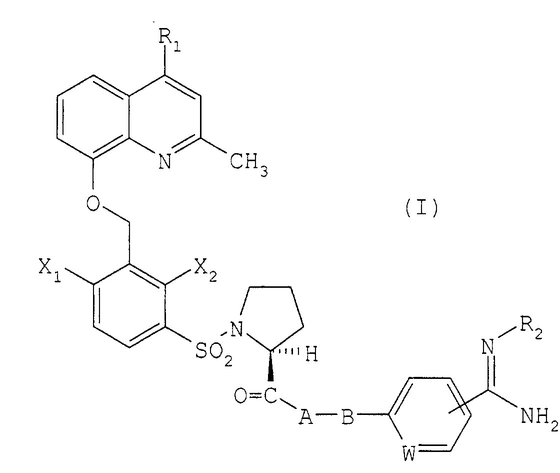 dans laquelle :
R₁ représente un atome d'hydrogène ou un groupe alkyle en C₁-C₃ à chaîne hydrocarbonée linéaire ou ramifiée,
X₁ et X₂ représentent le même atome d'halogène,
A représente un groupe -N(R₃)-(CH₂)ₙ-.
B représente une liaison simple,
W représente CH,
R₂ représente un atome d'hydrogène,
R₃ représente H ou CH₃, et
n représente 0, 1, 2 ou 3 ; et
(5°) si nécessaire, faire réagir le composé de formule I, ainsi obtenu, sous forme de base libre avec un acide minéral ou organique pour obtenir le sel d'addition d'acide correspondant ;

4. Procédé de préparation d'un composé de formule I **caractérisé en ce qu'**il comprend les étapes consistant à :
(1°) faire réagir un composé de formule : 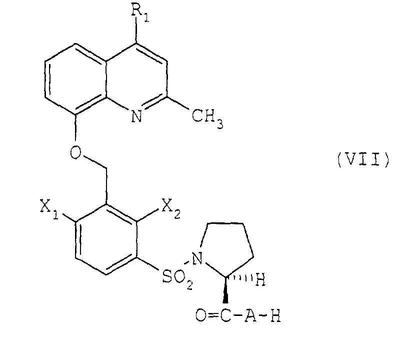 dans laquelle :
R₁ représente un atome d'hydrogène ou un groupe alkyle en C₁-C₃, à chaîne hydrocarbonée linéaire ou ramifiée,
X₁ et X₂ représentent le même halogène,
A représente un groupe, 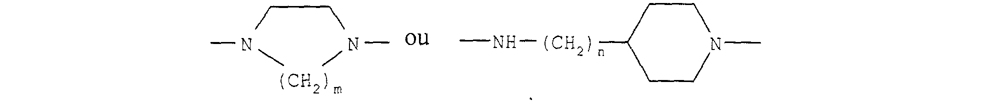
où n représente 0, 1, 2 ou 3, et m représente 2,
avec un composé de formule VIII : 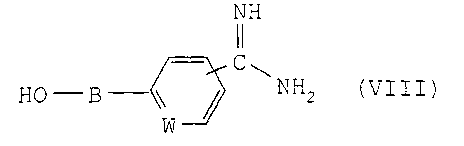 dans laquelle B représente -CO-, -CO-CH₂- ou -CO-CH₂-O-,
selon des conditions analogues à celles de l'étape (1°) de la variante A ci-dessus, pour obtenir un composé de formule : 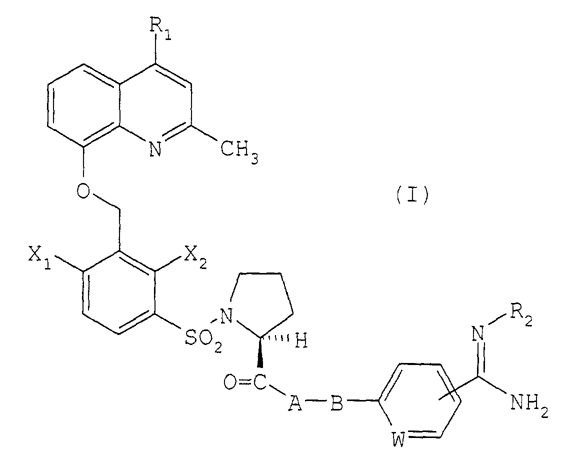 dans laquelle R₁, X₁, X₂ conservent la même signification que ci-dessus,
A représente un groupe 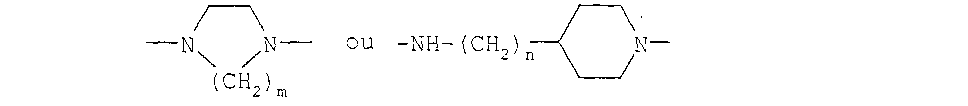
B représente un groupe -CO-, -CO-CH₂-, ou -CO-CH₂-O-
m représente 2,
n représente 0, 1, 2 ou 3,
W représente CH, et
R₂ représente un atome d'hydrogène ;
(2°) si nécessaire, faire réagir le composé de formule I, ainsi obtenu, avec un acide pour obtenir le sel d'addition d'acide correspondant ;

5. Composition thérapeutique **caractérisée en ce qu'**elle renferme, en association avec un excipient physiologiquement acceptable, au moins un composé choisi parmi l'ensemble constitué par les composés de formule I et leurs sels d'addition non-toxiques selon la revendication 1.

6. Utilisation d'une substance agoniste d'un récepteur de la bradykinine et des hormones analogues, ladite utilisation étant **caractérisée en ce que** l'on fait appel à une substance agoniste du récepteur B₂ de la bradykinine et choisie parmi l'ensemble constitué par les composés de formule I et leurs sels d'addition non toxiques selon la revendication 1, pour l'obtention d'un médicament destiné à une utilisation en thérapeutique vis-à-vis d'états pathologiques impliquant la bradykinine ou ses homologues.

7. Utilisation selon la revendication 6, d'une substance choisie parmi l'ensemble constitué par les composés de formule I et leurs sels d'addition non toxiques selon la revendication 1, pour l'obtention d'un médicament destiné à une utilisation en thérapeutique pour le traitement d'états douloureux.

8. Utilisation selon la revendication 6, d'une substance choisie parmi l'ensemble constitué par les composés de formule I et leurs sels d'addition non toxiques selon la revendication 1, pour l'obtention d'un médicament destiné à une utilisation en thérapeutique pour le traitement d'états inflammatoires.

9. Utilisation d'un composé de formule I ou l'un de ses sels d'addition selon la revendication 1 en tant que réactif pharmacologique.

## Patentansprüche

1. N-Benzolsulfonyl-(L)-prolin-Derivat, **dadurch gekennzeichnet, dass** es aus der Gesamtheit ausgewählt ist, die aus
a) Verbindungen mit der Formel 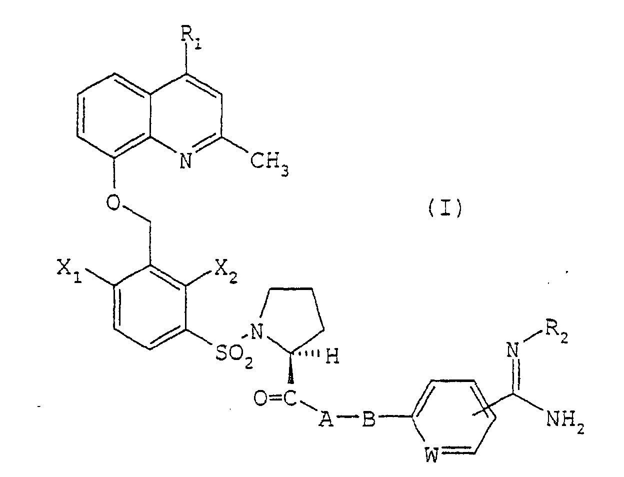 in welcher
X₁ und X₂ dasselbe Halogenatom bedeuten,
R₁ ein Wasserstoffatom oder eine C₁- bis C₃-Alkylgruppe mit geradkettiger oder verzweigter Kohlenwasserstoffkette,
R₂ ein Wasserstoffatom,
A eine Gruppe 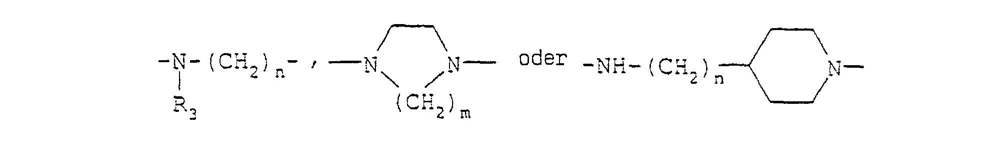
B eine Einfachbindung, -CO-, -CO-CH₂- oder -CO-CH₂-O-,
m 2,
n 0, 1, 2 oder 3,
R₃ ein Wasserstoffatom oder eine Methylgruppe und
W CH bedeutet,
wobei sich die Amidingruppe C(=NR₂)NH₂ am aromatischen Kern in 2-, 3- oder 4-Stellung befindet, und
b) ihren Additionssalzen besteht.

2. Verbindung nach Anspruch 1, **dadurch gekennzeichnet, dass** in der Formel I X₁ und X₂ Chloratome bedeuten.

3. Verfahren zur Herstellung einer Verbindung mit der Formel I, **dadurch gekennzeichnet, dass** es die Stufen, die aus
(1.) 2 bis 50 Stunden langes Umsetzen einer Säure mit der Formel 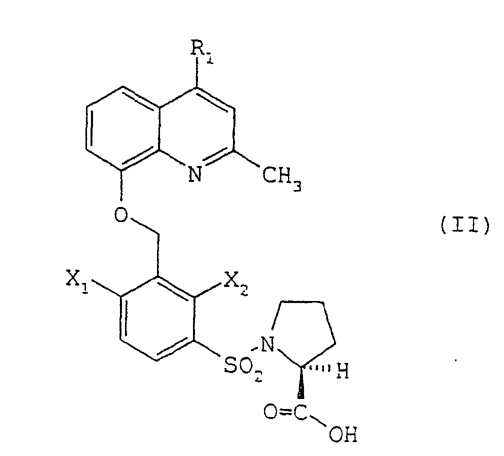 in welcher
R₁ ein Wasserstoffatom oder eine C₁- bis C₃-Alkylgruppe mit geradkettiger oder verzweigter Kohlenwasserstoffkette bedeutet und
X₁ und X₂ dasselbe Halogen bedeuten,
mit einem Amin mit der Formel 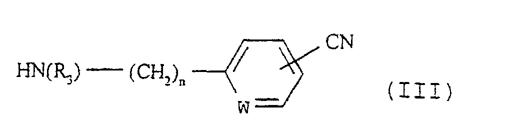 in welcher R₃ ein Wasserstoffatom oder eine Methylgruppe und
n 0, 1, 2 oder 3 und W CH bedeutet,
in einem Lösungsmittel und in Gegenwart von Aktivatoren, die üblicherweise für die Schaffung von Peptidbindungen verwendet werden, bei einer Temperatur in der Nähe der Umgebungstemperatur, um eine Verbindung mit der Formel 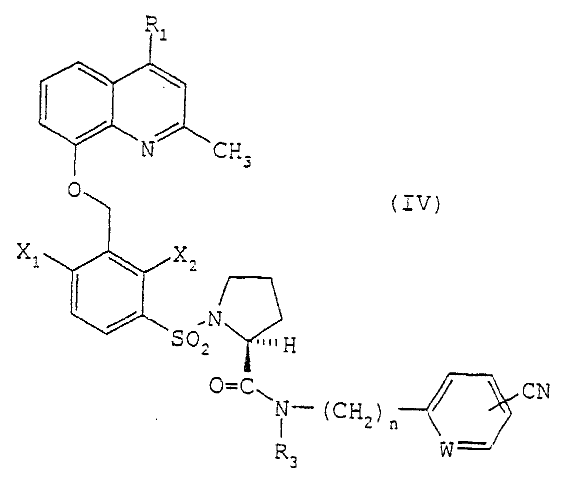 zu erhalten, in welcher R₁, R₃, X₁, X₂, n und W dieselbe Bedeutung wie zuvor beibehalten,
(2.) 2 bis 40 Stunden langes Umsetzen der so erhaltenen Verbindung mit der Formel IV mit Schwefelwasserstoff im Überschuß in einem wasserfreien Lösungsmittel in Gegenwart von Triethylamin bei einer Temperatur von 0 bis 40 °C, um eine Verbindung mit der Formel 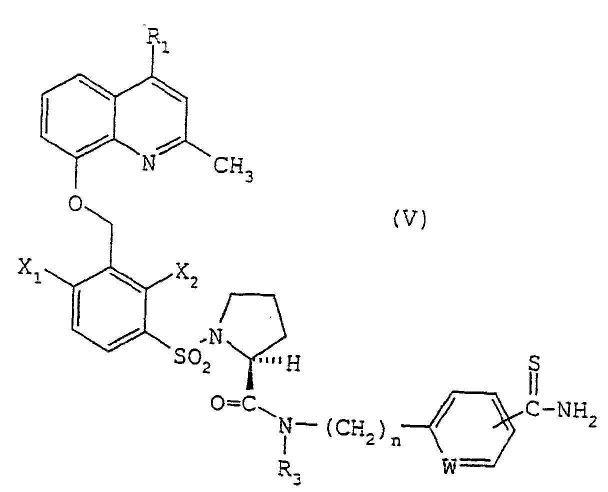 zu erhalten, in welcher R₁, R₃, X₁, X₂, n und W dieselbe Bedeutung wie zuvor beibehalten,
(3.) 1 bis 5 Stunden langes Umsetzen der so erhaltenen Verbindung mit der Formel V mit einem Methylierungsmittel im Überschuß in einem Lösungsmittel bei einer Temperatur in der Nähe der Siedetemperatur des Reaktionsmediums, gegebenenfalls unter Überdruck, um eine Verbindung mit der Formel 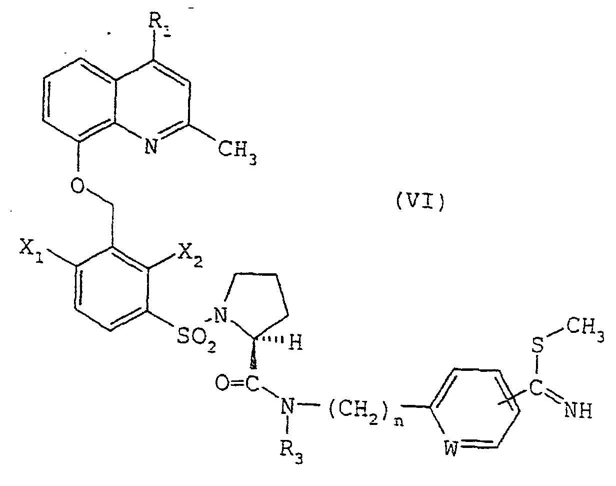 oder eines ihrer Additionssalze zu erhalten,
worin R₁, R₃, X₁, X₂, n und W dieselbe Bedeutung wie zuvor beibehalten,
(4.) 1 bis 10 Stunden langes Umsetzen der so erhaltenen Verbindung mit der Formel VI mit einem Ammoniumsalz in einem Lösungsmittel bei einer Temperatur von Umgebungstemperatur bis 100 °C, um eine Verbindung mit der Formel I 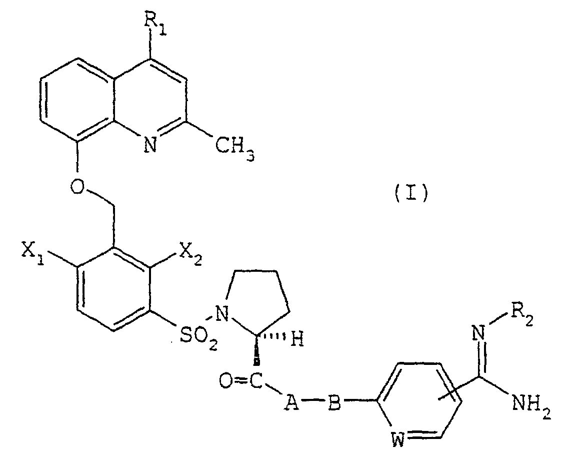 zu erhalten, in welcher
R₁ ein Wasserstoffatom oder eine C₁- bis C₃-Alkylgruppe mit geradkettiger oder verzweigter Kohlenwasserstoffkette bedeutet,
X₁ und X₂ dasselbe Halogenatom bedeuten,
A eine -N(R₃) - (CH₂)ₙ-Gruppe,
B eine Einfachbindung,
W CH,
R₂ ein Wasserstoffatom,
R₃ H oder CH₃ und
n 0, 1, 2 oder 3 bedeutet, und
(5.) erforderlichenfalls Umsetzen der so in Form einer freien Base erhaltenen Verbindung mit der Formel I mit einer anorganischen oder organischen Säure, um das entsprechende Säureadditionssalz zu erhalten,
bestehen, umfasst.

4. Verfahren zur Herstellung einer Verbindung mit der Formel I, **dadurch gekennzeichnet, dass** es die Stufen, die aus
(1.) Umsetzen einer Verbindung mit der Formel 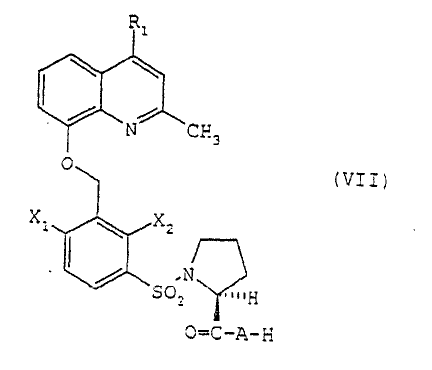 in welcher
R₁ ein Wasserstoffatom oder eine C₁- bis C₃-Alkylgruppe mit geradkettiger oder verzweigter Kohlenwasserstoffkette bedeutet,
X₁ und X₂ dasselbe Halogenatom bedeuten und
A eine Gruppe 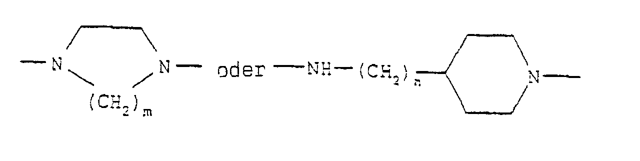
bedeutet, worin n 0, 1, 2 oder 3 und m 2 bedeutet, mit einer Verbindung mit der Formel VIII 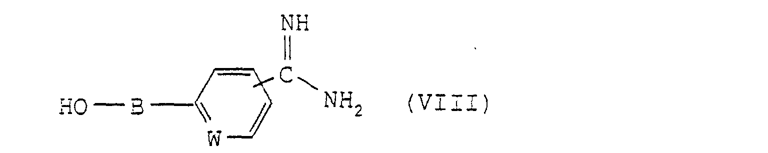 in welcher B -CO-, -CO-CH₂- oder -CO-CH₂-O- bedeutet, unter Bedingungen, die denen der Stufe (1.) obiger Abwandlung A analog sind, um eine Verbindung mit der Formel 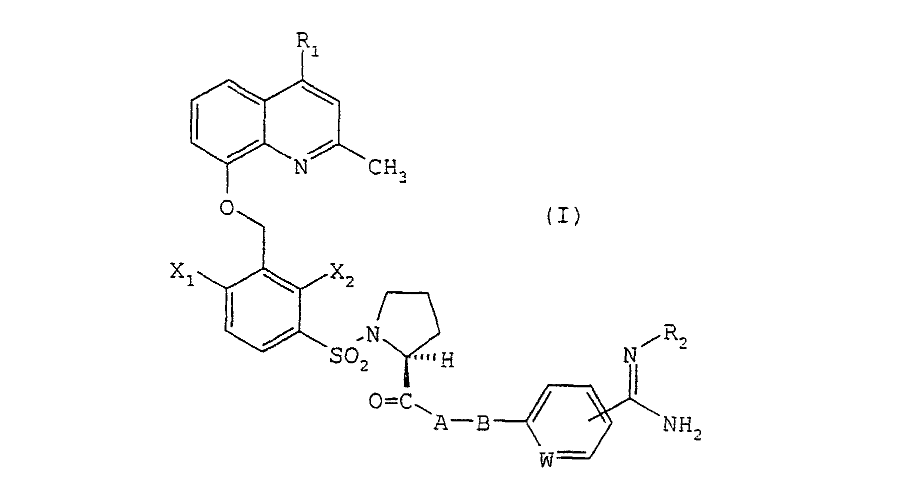 zu erhalten, in welcher R₁, X₁, und X₂ dieselbe Bedeutung wie zuvor beibehalten, wobei
A eine Gruppe 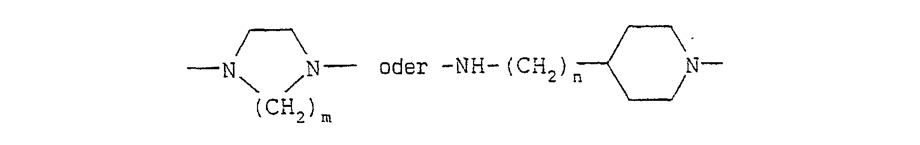
B eine -CO-, -CO-CH₂- oder -CO-CH₂-O-Gruppe,
m 2,
n 0, 1, 2 oder 3,
W CH und
R₂ ein Wasserstoffatom bedeutet, und
(2.) erforderlichenfalls Umsetzen der so erhaltenen Verbindung mit der Formel I mit einer Säure, um das entsprechende Säureadditionssalz zu erhalten,
bestehen, umfasst.

5. Therapeutische Zusammensetzung, **dadurch gekennzeichnet, dass** sie zusammen mit einem physiologisch verträglichen Arzneimittelträger mindestens eine Verbindung enthält, die aus der Gesamtheit ausgewählt ist, die von den Verbindungen mit der Formel I und deren nichttoxischen Additionssalzen nach Anspruch 1 gebildet wird.

6. Verwendung einer Substanz, die an einem Rezeptor für Bradykinin und analoge Hormone ein Agonist ist, wobei die Verwendung **dadurch gekennzeichnet ist, dass** auf eine Substanz zurückgegriffen wird, die am Bradykinin-B2-Rezeptor ein Agonist und aus der Gesamtheit ausgewählt ist, die von den Verbindungen mit der Formel I und deren nichttoxischen Additionssalzen nach Anspruch 1 gebildet wird, um ein Arzneimittel zu erhalten, das für eine therapeutische Verwendung bei Erkrankungen vorgesehen ist, an denen Bradykinin oder dessen Homologen beteiligt sind.

7. Verwendung nach Anspruch 6 einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei der Behandlung von Schmerzzuständen vorgesehen ist.

8. Verwendung nach Anspruch 6 einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei der Behandlung von Entzündungszuständen vorgesehen ist.

9. Verwendung einer Verbindung mit der Formel I oder eines ihrer Additionssalze nach Anspruch 1 als pharmazeutischer Wirkstoff.

## Claims

1. Compound derived from N-benzenesulfonyl-(L)-proline, **characterized in that** it is selected from the group consisting of:
(i) the compounds of formula 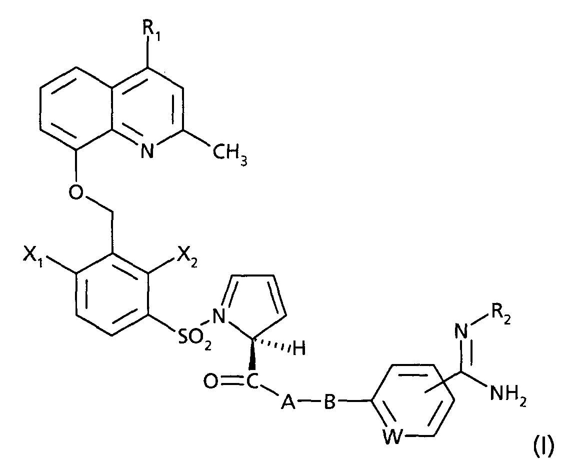 in which:
X₁ and X₂ are the same halogen atom,
R₁ is a hydrogen atom or a C₁-C₃-alkyl group with a linear or branched hydrocarbon chain,
R₂ is a hydrogen atom,
A is a group 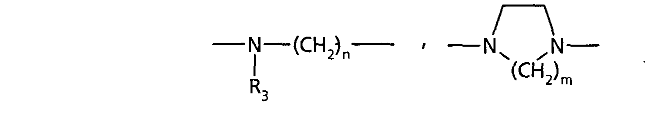 or 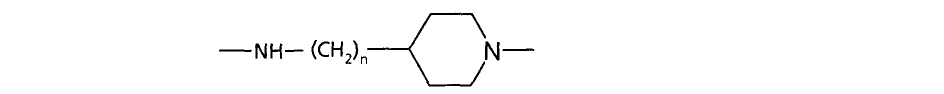
B is a single bond, -CO-, -CO-CH₂- or -CO-CH₂-O-,
m is 2,
n is 0, 1, 2 or 3,
R₃ is a hydrogen atom or a methyl group, and
W is CH,
the amidine group C(=NR₂)NH₂ being in the 2-, 3- or 4-position on the aromatic ring; and
(ii) their addition salts.

2. Compound according to claim 1, **characterized in that** X₁ and X₂ in formula 1 are each the chlorine atom.

3. Process for the preparation of a compound of formula I, **characterized in that** it comprises the steps which consist in:
1) reacting an acid of formula 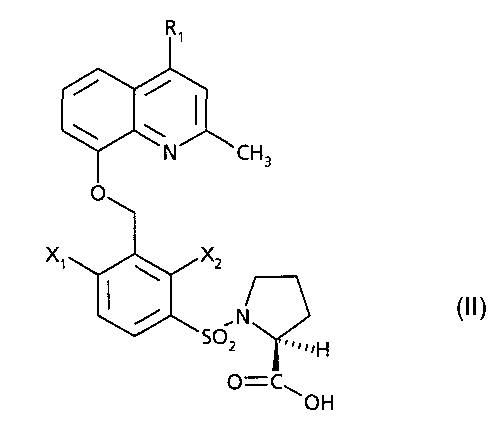 in which:
R₁ is a hydrogen atom or a C₁-C₃-alkyl group with a linear or branched hydrocarbon chain, and
X₁ and X₂ are the same halogen,
with an amine of formula III 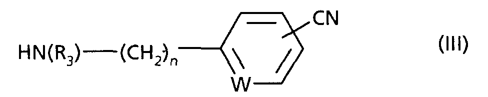 in which R₃ is a hydrogen atom or a methyl group,
n is 0, 1, 2 or 3, and W is CH,
in a solvent, in the presence of activators commonly used to create bonds of the peptide type, at a temperature close to room temperature, for 2 to 50 hours, to give a compound of formula 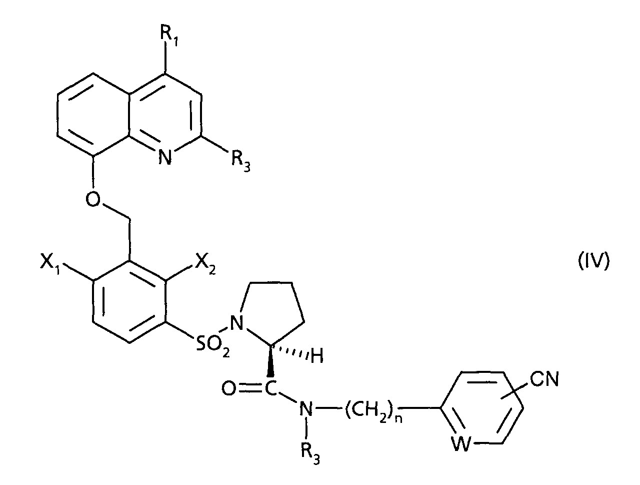 in which R₁, R₃, X₁, X₂, n and W are as defined above;
2) reacting the resulting compound of formula IV with excess hydrogen sulfide, in an anhydrous solvent, in the presence of triethylamine, at a temperature between 0 and 40° C, for 2 to 40 hours, to give a compound of formula 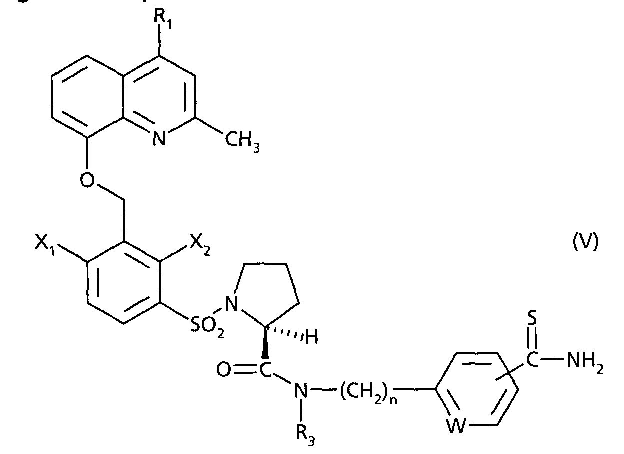 in which R₁, R₃, X₁, X₂, n and W are as defined above;
3) reacting the resulting compound of formula V with an excess of a methylating agent, in a solvent, at a temperature close to the boiling point of the reaction medium, optionally under a pressure above atmospheric pressure, for 1 to 5 hours, to give a compound of formula 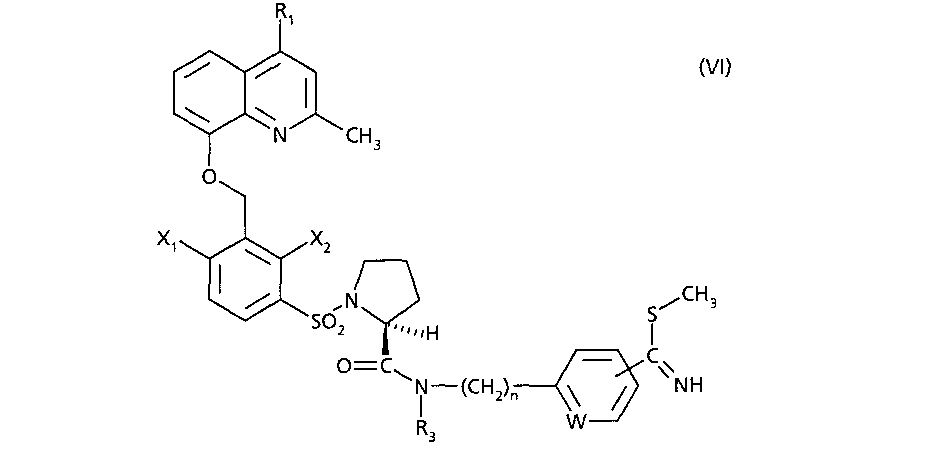 or one of its addition salts,
in which R₁, R₃, X₁, X₂, n and W are as defined above;
4) reacting the resulting compound of formula VI with an ammonium salt, in a solvent, at a temperature between room temperature and 100° C, for 1 to 10 hours, to give a compound of formula I: 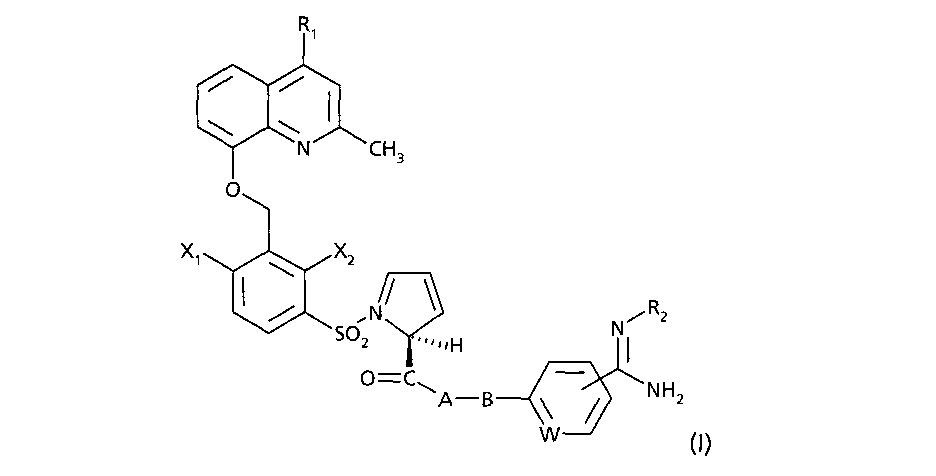 in which:
R₁ is a hydrogen atom or a C₁-C₃-alkyl group with a linear or branched hydrocarbon chain,
X₁ and X₂ are the same halogen atom,
A is a group -N(R₃)-(CH₂)ₙ-,
B is a single bond,
W is CH,
R₂ is a hydrogen atom,
R₃ is H or CH₃, and
n is 0, 1, 2 or 3 ; and
5) if necessary, reacting the resulting compound of formula I, in the form of a free base, with a mineral or organic acid to give a corresponding acid addition salt.

4. Process for the preparation of a compound of formula I, **characterized in that** it comprises the steps which consist in :
1) reacting a compound of formula 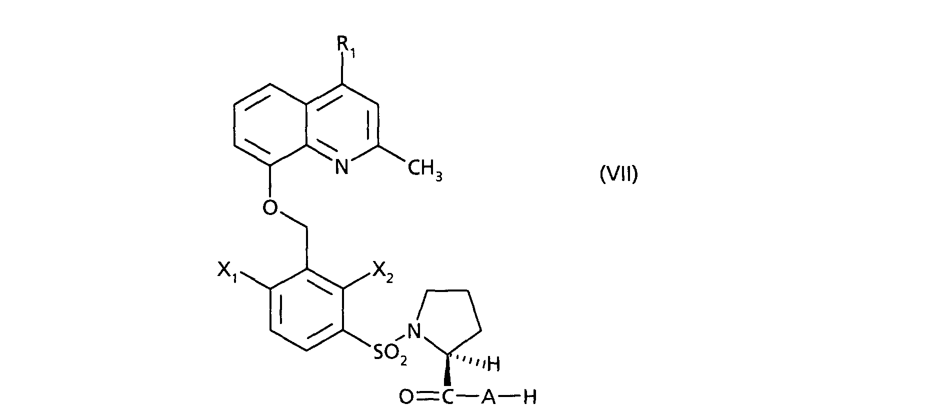 in which:
R₁ is a hydrogen atom or a C₁-C₃-alkyl group with a linear or branched hydrocarbon chain,
X₁ and X₂ are the same halogen,
A is a group 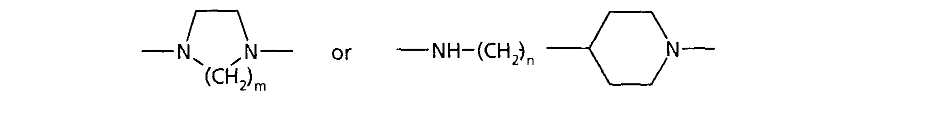
in which n is 0, 1, 2 or 3, and m is 2,
with a compound of formula VIII: 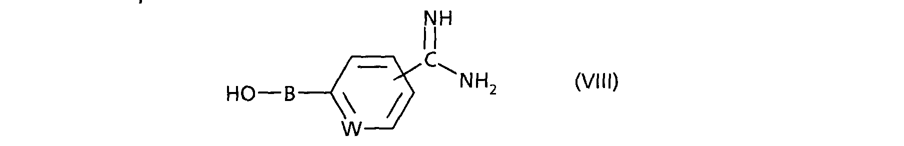 in which B is -CO-, -CO-CH₂- or -CO-CH₂-O-,
under conditions analogous to those of step 1) in the embodiment A above, to give a compound of formula 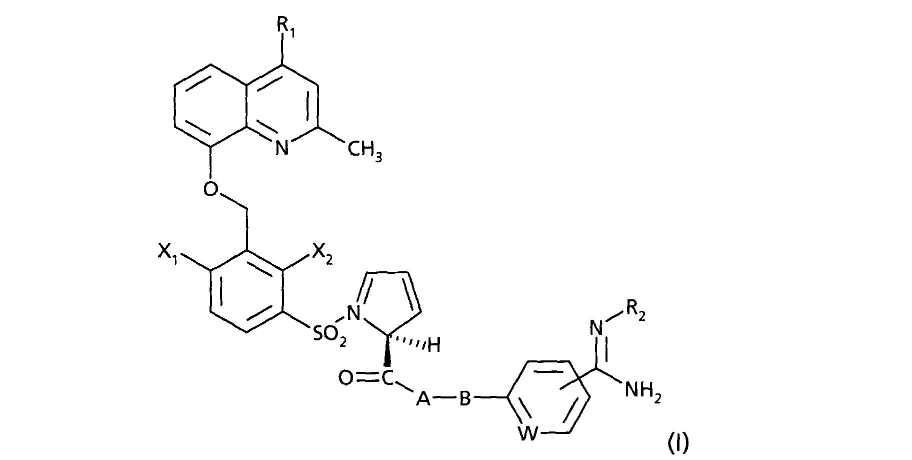 in which R₁, X₁ and X₂ are as defined above,
A is a group 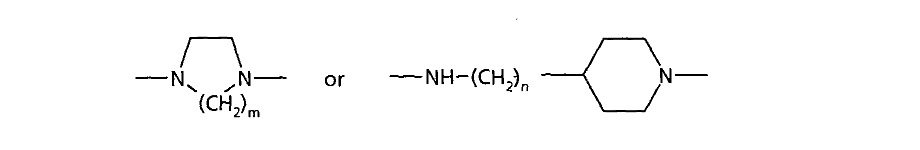
B is a group -CO-, -CO-CH₂- or -CO-CH₂-O-,
m is 2,
n is 0, 1, 2, or 3
W is CH, and
R₂ is a hydrogen atom.
2) if necessary, reacting the resulting compound of formula I with an acid to give a corresponding acid addition salt.

5. Therapeutic composition, **characterized in that** it contains, in association with a physiologically acceptable excipient, at least one compound selected from the group consisting of the compounds of formula I and their non-toxic addition salts according to claim 1.

6. Use of an antagonist of a receptor of bradykinin and analogous hormones, said use being **characterized in that** a bradykinin B₂ receptor antagonist selected from the group consisting of the compounds of formula I and their non-toxic addition salts according to claim 1 is employed for obtaining a drug intended for use in therapeutics to combat pathological conditions involving bradykinin or its homologs.

7. Use according to claim 6 of a substance selected from the group consisting of the compounds of formula I and their non-toxic addition salts according to claim 1 for obtaining a drug intended for use in therapeutics for the treatment of painful states.

8. Use according to claim 6 of a substance selected from the group consisting of the compounds of formula I and their non-toxic addition salts according to claim 1 for obtaining a drug intended for use in therapeutics for the treatment of inflammatory states.

9. Use of a compound of formula I or one of its addition salts according to claim 1 as a pharmacological reagent.
